# EUROPEAN PATENT SPECIFICATION

(11) **EP 0 452 925 B1**
(45) Date of publication and mention of the grant of the patent: **09.02.2000**
(21) Application number: 91106217.2
(22) Date of filing: 18.04.1991
(51) Int. Cl.: C12N 15/12, C07K 14/435

(54) **Isolated DNA coding for spider silk protein, a replicable vector and a transformed cell containing the isolated DNA, and products thereof**
Für Spinnenseideprotein kodierende DNS, DNS enthaltender Vektor, transformierte Zelle und Produkte davon
ADN codante pour la protéine de la soie d'araignée, vecteur et cellule transformée la contenant, et produits

(30) Priority: 20.04.1990 US 511792
(43) Date of publication of application: 23.10.1991
(73) Proprietor: THE UNIVERSITY OF WYOMING, Laramie, Wyoming 82071-3355 (US)
(72) Inventor: Lewis, Randolph V., Laramie WY 82070 (US); Xu, Ming Dept. Medical Physiology/Biochemistry, Calgary Alberta T2N 3YT (CA); Hinman, Michael, Laramie WY 82070 (US)
(74) Representative: Benedum, Ulrich Max, Dr.

(56) References cited:
- EP-A- 0 294 979
- WO-A-91/16351
- CHEMICAL ABSTRACTS, vol. 111, no. 17, 23 October 1989, Columbus, Ohio, US; abstract no. 147880F, R. V. LEWIS: 'Cloning and structure of different types of spider silk' page 182 ;column L & GOV. REP. ANNOUNCE. INDEX (U.S.); ABSTR. NO. 926.437 vol. 89, no. 10, 1989, WASHINGTON, D.C., US
- ACTA ZOOL. FENNICA vol. 190, 21 December 1990, HELSINKI, SF pages 243 - 248; S. J. LOMBARDI AND D. L. KAPLAN: 'The Nephila clavipes major ampullate gland silk protein: amino acid composition and detection of silk gene-related nucleic acids in the genome'
- POLYMER PREPRINTS vol. 31, no. 1, January 1990, NEWARK, NJ US pages 195 - 196; S. J. LOMBARDI AND D. L. KAPLAN: 'ISOLATION, CLONING, AND PHYSICOCHEMICAL CHARACTERIZATION OF SPIDER SILK FROM THE GOLDEN ORB-WEAVER, Nephila clavipes'
- DISS. ABSTR. INT. B vol. 52, no. 2, 1991, ANN ARBOR, MI US page 669; M. XU: 'Sequence of a partial cDNA clone from a major protein of Nephila clavipes dragline silk' & DISSERTATION, UNIVERSITY OF WYOMING, US, 1990; CHAIRMAN: RANDOLPH V. LEWIS
- PROCEEDINGS OF THE NATIONAL ACADEMY OF SCIENCES OF USA. vol. 87, September 1990, WASHINGTON US pages 7120 - 7124; M. XU AND R. V. LEWIS: 'Structure of a protein superfiber: spider dragline silk'
- CHEMICAL ABSTRACTS, vol. 114, no. 15, 15 April 1991, Columbus, Ohio, US; abstract no. 137094A, R. V. LEWIS ET AL.: 'Cloning and structure of different types of spider silk' page 182 ;column L & (UNIV. WYOMING, LARAMIE, WY USA), REPORT 1989, ORDER NO. AD-A216217. AVAIL. NTIS. FROM GOV. REP. ANNOUNCE. INDEX (U.S.); ABSTR. NO. 018.084 vol.90. no. 8, 1990, WASHINGTON, D.C., US

SEQUENCE LISTING

(1) GENERAL INFORMATION:

(i) APPLICANT: Lewis, Randolph V.
Xu, Ming

(ii) TITLE OF INVENTION: ISOLATED cDNA CODING FOR SPIDER SILK
PROTEIN, A REPLICABLE VECTOR AND A TRANSFORMED CELL
CONTAINING THE ISOLATED cDNA, AND PRODUCTS THEREOF

(iii) NUMBER OF SEQUENCES: 4

(2) INFORMATION FOR SEQ ID NO:1:

(i) SEQUENCE CHARACTERISTICS:
(A) LENGTH: 2338 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(vi) ORIGINAL SOURCE:
(A) ORGANISM: Nephilia clavipes

(ix) FEATURE:
(A) NAME/KEY: CDS
(B) LOCATION: 1..2154
(D) OTHER INFORMATION: /product= "Nephila clavipes
dragline silk protein"

(x) PUBLICATION INFORMATION:
(A) AUTHORS: Xu, Ming
Lewis, Randolph V.
(B) TITLE: Structure of a protein superfiber: Spider
drafline silk
(C) JOURNAL: Proc. Natl. Acad. Sci. U.S.A.
(D) VOLUME: 87
(F) PAGES: 7120-7124
(G) DATE: Sept.-1990
(K) RELEVANT RESIDUES IN SEQ ID NO:1: FROM 1 TO 2338

(xi) SEQUENCE DESCRIPTION: SEQ ID NO:1:

CAA GGG GCA GGT GCA GCA GCA GCA GCA GCT GGA GGT GCC GGA CAA GGA 48
Gln Gly Ala Gly Ala Ala Ala Ala Ala Ala Gly Gly Ala Gly Gln Gly
1               5                   10                  15

GGA TAT GGA GGT CTT GGT GGA CAA GGA GCT GGT CAA GGT GGA TAT GGA 96
Gly Tyr Gly Gly Leu Gly Gly Gln Gly Ala Gly Gln Gly Gly Tyr Gly
            20                  25                  30

GGT CTT GGT GGA CAA GGT GCC GGA CAA GGA GCT GGT GCA GCC GCC GCA 144
Gly Leu Gly Gly Gln Gly Ala Gly Gln Gly Ala Gly Ala Ala Ala Ala
        35                  40                  45

GCA GCA GCT GGT GGT GCC GGA CAA GGA GGA TAT GGA GGT CTT GGA AGC 192
Ala Ala Ala Gly Gly Ala Gly Gln Gly Gly Tyr Gly Gly Leu Gly Ser
    50                  55                  60

CAA GGT GCT GGA CGA GGT GGA CAA GGA GCT GGA GCA GCC GCT GCA GCT 240
Gln Gly Ala Gly Arg Gly Gly Gln Gly Ala Gly Ala Ala Ala Ala Ala
65                  70                  75                  80

GCG GGT GGT GCC GGA CAA GGA GGT TAT GGA GGT CTT GGA AGT CAA GGT 288
Ala Gly Gly Ala Gly Gln Gly Gly Tyr Gly Gly Leu Gly Ser Gln Gly
                85                  90                  95

GCA GGA CGA GGT GGA TTA GGT GGA CAA GGG GCA GGT GCA GCA GCC GCT 336
Ala Gly Arg Gly Gly Leu Gly Gly Gln Gly Ala Gly Ala Ala Ala Ala
            100                 105                 110

GCA GCA GCT GGA GGT GCC GGA CAA GGA GGA TAT GGA GGC CTT GGA AAC 384
Ala Ala Ala Gly Gly Ala Gly Gln Gly Gly Tyr Gly Gly Leu Gly Asn
        115                 120                 125

CAA GGT GCT GGA CGA GGT GGA CAA GGT GCA GCA GCA GCA GCA GCT GGA 432
Gln Gly Ala Gly Arg Gly Gly Gln Gly Ala Ala Ala Ala Ala Ala Gly
    130                 135                 140

GGT GCT GGA CAA GGA GGA TAT GGA GGT CTT GGA AGC CAA GGT GCA GGA 480
Gly Ala Gly Gln Gly Gly Tyr Gly Gly Leu Gly Ser Gln Gly Ala Gly
145                 150                 155                 160

CGA GGT GGA TTA GGT GGA CAA GGT GCA GGT GCA GCA GCA GCA GCA GCC 528
Arg Gly Gly Leu Gly Gly Gln Gly Ala Gly Ala Ala Ala Ala Ala Ala
                165                 170                 175

GGA GGT GCT GGA CAA GGC GGA TAC GGT GGT CTT GGT GGA CAA GGT GCC 576
Gly Gly Ala Gly Gln Gly Gly Tyr Gly Gly Leu Gly Gly Gln Gly Ala
            180                 185                 190

GGA CAA GGA GGC TAT GGA GGA CTT GGA AGC CAA GGT GCT GGA CGA GGA 624
Gly Gln Gly Gly Tyr Gly Gly Leu Gly Ser Gln Gly Ala Gly Arg Gly
        195                 200                 205

GGA TTA GGT GGA CAA GGT GCA GGT GCA GCA GCA GCA GCA GCA GCT GGA 672
Gly Leu Gly Gly Gln Gly Ala Gly Ala Ala Ala Ala Ala Ala Ala Gly
    210                 215                 220

GGT GCC GGA CAA GGA GGA CTA GGT GGA CAA GGT GCT GGA CAA GGA GCT 720
Gly Ala Gly Gln Gly Gly Leu Gly Gly Gln Gly Ala Gly Gln Gly Ala
225                 230                 235                 240

[?]GA GCA TCC GCT GCA GCA GCT GGT GGT GCC GGA CAA GGA GGA TAT GGA 768
Gly Ala Ser Ala Ala Ala Ala Gly Gly Ala Gly Gln Gly Gly Tyr Gly
                245                 250                 255

GGT CTT GGA AGC CAA GGT GCT GGA CGA GGT GGA GAA GGT GCA GGC GCA 816
Gly Leu Gly Ser Gln Gly Ala Gly Arg Gly Gly Glu Gly Ala Gly Ala
            260                 265                 270

GCC GCA GCA GCA GCC GGA GGT GCT GGA CAA GGA GGA TAC GGT GGT CTT 864
Ala Ala Ala Ala Ala Gly Gly Ala Gly Gln Gly Gly Tyr Gly Gly Leu
        275                 280                 285

GGT GGA CAA GGT GCC GGA CAA GGA GGC TAT GGA GGA CTT GGA AGC CAA 912
Gly Gly Gln Gly Ala Gly Gln Gly Gly Tyr Gly Gly Leu Gly Ser Gln
    290                 295                 300

GGT GCT GGA CGA GGA GGA TTA GGT GGA CAA GGT GCA GGT GCA GCA GCA 960
Gly Ala Gly Arg Gly Gly Leu Gly Gly Gln Gly Ala Gly Ala Ala Ala
305                 310                 315                 320

GCT GGA GGT GCC GGG CAA GGA GGA CTA GGT GGA CAA GGT GCT GGA CAA 1008
Ala Gly Gly Ala Gly Gln Gly Gly Leu Gly Gly Gln Gly Ala Gly Gln
                325                 330                 335

GGA GCT GGA GCA GCC GCT GCA GCA GCT GGT GGT GCC GGA CAA GGA GGA 1056
Gly Ala Gly Ala Ala Ala Ala Ala Ala Gly Gly Ala Gly Gln Gly Gly
            340                 345                 350

TAT GGA GGT CTT GGA AGC CAA GGT GCA GGA CGA GGT GGA TTA GGT GGA 1104
Tyr Gly Gly Leu Gly Ser Gln Gly Ala Gly Arg Gly Gly Leu Gly Gly
        355                 360                 365

[?]AA [?]GG GCA GGT GCA GTA GCC GCT GCA GCA GCT GGA GGT GCC GGA CAA 1152
[?]ln Gly Ala Gly Ala Val Ala Ala Ala Ala Ala Gly Gly Ala Gly Gln
370 375 380

GGA GGA TAT GGA GGT CTT GGA AGC CAA GGT GCT GGA CGA GGT GGA CAA 1200
Gly Gly Tyr Gly Gly Leu Gly Ser Gln Gly Ala Gly Arg Gly Gly Gln
385 390 395 400

GGA GCT GGA GCA GCC GCT GCA GCA GCT GGT GGT GCC GGA CAA AGA GGT 1248
Gly Ala Gly Ala Ala Ala Ala Ala Ala Gly Gly Ala Gly Gln Arg Gly
405 410 415

TAT GGA GGT CTT GGA AAT CAA GGT GCA GGA CGA GGT GGA TTA GGT GGA 1296
Tyr Gly Gly Leu Gly Asn Gln Gly Ala Gly Arg Gly Gly Leu Gly Gly
420 425 430

CAA GGG GCA GGT GCA GCA GCC GCT GCA GCA GCT GGA GGT GCC GGA CAA 1344
Gln Gly Ala Gly Ala Ala Ala Ala Ala Ala Ala Gly Gly Ala Gly Gln
435 440 445

[?]GA GGA TAT GGA GGC CTT GGA AAC CAA GGT GCT GGA CGA GGT GGA CAA 1392
Gly Gly Tyr Gly Gly Leu Gly Asn Gln Gly Ala Gly Arg Gly Gly Gln
450 455 460

GGT GCA GCA GCA GCA GCT GGA GGT GCC GGA CAA GGA GGA TAT GGA GGT 1440
Gly Ala Ala Ala Ala Ala Gly Gly Ala Gly Gln Gly Gly Tyr Gly Gly
465 470 475 480

CTT GGA AGC CAA GGT GCT GGA CGA GGT GGA CAA GGT GCA GGC GCA GCC 1488
Leu Gly Ser Gln Gly Ala Gly Arg Gly Gly Gln Gly Ala Gly Ala Ala
485 490 495

GCA GCA GCA GCC GTA GGT GCT GGA CAA GAA GGA ATA CGT GGA CAA GGT 1536
Ala Ala Ala Ala Val Gly Ala Gly Gln Glu Gly Ile Arg Gly Gln Gly
500 505 510

GCC GGA CAA GGA GGC TAT GGA GGA CTT GGA AGC CAA GGT TCT GGT CGA 1584
Ala Gly Gln Gly Gly Tyr Gly Gly Leu Gly Ser Gln Gly Ser Gly Arg
515 520 525

GGA GGA TTA GGT GGA CAA GGT GCA GGT GCA GCA GCA GCA GCA GCT GGA 1632
Gly Gly Leu Gly Gly Gln Gly Ala Gly Ala Ala Ala Ala Ala Ala Gly
530 535 540

GGT GCT GGA CAA GGA GGA TTA GGT GGA CAA GGT GCT GGA CAA GGA GCT 1680
Gly Ala Gly Gln Gly Gly Leu Gly Gly Gln Gly Ala Gly Gln Gly Ala
545 550 555 560

GGA GCA GCC GCT GCA GCA GCT GGT GGT GTT AGA CAA GGA GGA TAT GGA 1728
Gly Ala Ala Ala Ala Ala Ala Gly Gly Val Arg Gln Gly Gly Tyr Gly
565 570 575

[?]GT CTT GGA AGC CAA GGT GCT GGA CGA GGT GGA CAA GGT GCA GGC GCA 1776
[?]ly Leu Gly Ser Gln Gly Ala Gly Arg Gly Gly Gln Gly Ala Gly Ala
580 585 590

GCC GCA GCA GCA GCC GGA GGT GCT GGA CAA GGA GGA TAT GGT GGT CTT 1824
Ala Ala Ala Ala Ala Gly Gly Ala Gly Gln Gly Gly Tyr Gly Gly Leu
595 600 605

GGT GGA CAA GGT GTT GGC CGA GGT GGA TTA GGT GGA CAG GGT GCA GGC 1872
Gly Gly Gln Gly Val Gly Arg Gly Gly Leu Gly Gly Gln Gly Ala Gly
610 615 620

GCA GCG GCA GCT GGT GGT GCT GGA CAA GGA GGA TAT GGT GGT GTT GGT 1920
Ala Ala Ala Ala Gly Gly Ala Gly Gln Gly Gly Tyr Gly Gly Val Gly
625 630 635 640

TCT GGG GCG TCT GCT GCC TCT GCA GCT GCA TCC CGG TTG TCT TCT CCT 1968
Ser Gly Ala Ser Ala Ala Ser Ala Ala Ala Ser Arg Leu Ser Ser Pro
645 650 655

[?]AA GCT AGT TCA AGA GTT TCA TCA GCT GTT TCC AAC TTG GTT GCA AGT 2016
Gln Ala Ser Ser Arg Val Ser Ser Ala Val Ser Asn Leu Val Ala Ser
660 665 670

GGT CCT ACT AAT TCT GCG GCC TTG TCA AGT ACA ATC AGT AAC GTG GTT 2064
Gly Pro Thr Asn Ser Ala Ala Leu Ser Ser Thr Ile Ser Asn Val Val
675 680 685

TCA CAA ATT GGC GCC AGC ATC CTG GTC TTT CTG GAT GTG ATG TCC TCA 2112
Ser Gln Ile Gly Ala Ser Ile Leu Val Phe Leu Asp Val Met Ser Ser
690 695 700

TTC AAG CTC TTC TCG AGG TTG TTT CTG CTC TTA TCC AGA TCT 2154
Phe Lys Leu Phe Ser Arg Leu Phe Leu Leu Leu Ser Arg Ser
705 710 715

TAGGTTCTTC CAGCATCGGC CAAGTTAACT ATGGTTCCGC TGGACAAGCC ACTCAGATCG 2214

TTGGTCAATC AGTTTATCAA GCCCTAGGTT AAATGTAAAA TCAAGAGTTG CTAAAACTTA 2274

ATGAACTCGG GCTGTTTATT TGTGTTAGGT TTTAAAATAT TTTCAATAAA TATTATGCAT 2334

ATAA 2338

(2) INFORMATION FOR SEQ ID NO:2:

(i) SEQUENCE CHARACTERISTICS:
(A) LENGTH: 718 amino acids
(B) TYPE: amino acid
(D) TOPOLOGY: linear

(ii) MOLECULE TYPE: protein

(xi) SEQUENCE DESCRIPTION: SEQ ID NO:2:

Gln Gly Ala Gly Ala Ala Ala Ala Ala Ala Gly Gly Ala Gly Gln Gly
1               5                   10                  15

Gly Tyr Gly Gly Leu Gly Gly Gln Gly Ala Gly Gln Gly Gly Tyr Gly
            20                  25                  30

Gly Leu Gly Gly Gln Gly Ala Gly Gln Gly Ala Gly Ala Ala Ala Ala
        35                  40                  45

Ala Ala Ala Gly Gly Ala Gly Gln Gly Gly Tyr Gly Gly Leu Gly Ser
    50                  55                  60

Gln Gly Ala Gly Arg Gly Gly Gln Gly Ala Gly Ala Ala Ala Ala Ala
65                  70                  75                  80

Ala Gly Gly Ala Gly Gln Gly Gly Tyr Gly Gly Leu Gly Ser Gln Gly
                85                  90                  95

Ala Gly Arg Gly Gly Leu Gly Gly Gln Gly Ala Gly Ala Ala Ala Ala
            100                 105                 110

Ala Ala Ala Gly Gly Ala Gly Gln Gly Gly Tyr Gly Gly Leu Gly Asn
        115                 120                 125

Gln Gly Ala Gly Arg Gly Gly Gln Gly Ala Ala Ala Ala Ala Ala Gly
    130                 135                 140

Gly Ala Gly Gln Gly Gly Tyr Gly Gly Leu Gly Ser Gln Gly Ala Gly
145                 150                 155                 160

Arg Gly Gly Leu Gly Gly Gln Gly Ala Gly Ala Ala Ala Ala Ala Ala
                165                 170                 175

Gly Gly Ala Gly Gln Gly Gly Tyr Gly Gly Leu Gly Gly Gln Gly Ala
            180                 185                 190

Gly Gln Gly Gly Tyr Gly Gly Leu Gly Ser Gln Gly Ala Gly Arg Gly
        195                 200                 205

Gly Leu Gly Gly Gln Gly Ala Gly Ala Ala Ala Ala Ala Ala Ala Gly
    210                 215                 220

Gly Ala Gly Gln Gly Gly Leu Gly Gly Gln Gly Ala Gly Gln Gly Ala
225                 230                 235                 240

Gly Ala Ser Ala Ala Ala Ala Gly Gly Ala Gly Gln Gly Gly Tyr Gly
                245                 250                 255

Gly Leu Gly Ser Gln Gly Ala Gly Arg Gly Gly Glu Gly Ala Gly Ala
            260                 265                 270

Ala Ala Ala Ala Ala Gly Gly Ala Gly Gln Gly Gly Tyr Gly Gly Leu
        275                 280                 285

[?]ly Gly Gln Gly Ala Gly Gln Gly Gly Tyr Gly Gly Leu Gly Ser Gln
    290                 295                 300

Gly Ala Gly Arg Gly Gly Leu Gly Gly Gln Gly Ala Gly Ala Ala Ala
305                 310                 315                 320

Ala Gly Gly Ala Gly Gln Gly Gly Leu Gly Gly Gln Gly Ala Gly Gln
                325                 330                 335

Gly Ala Gly Ala Ala Ala Ala Ala Ala Gly Gly Ala Gly Gln Gly Gly
            340                 345                 350

Tyr Gly Gly Leu Gly Ser Gln Gly Ala Gly Arg Gly Gly Leu Gly Gly
        355                 360                 365

Gln Gly Ala Gly Ala Val Ala Ala Ala Ala Ala Gly Gly Ala Gly Gln
    370                 375                 380

Gly Gly Tyr Gly Gly Leu Gly Ser Gln Gly Ala Gly Arg Gly Gly Gln
[?]5                 390                 395                 400

Gly Ala Gly Ala Ala Ala Ala Ala Ala Gly Gly Ala Gly Gln Arg Gly
                405                 410                 415

Tyr Gly Gly Leu Gly Asn Gln Gly Ala Gly Arg Gly Gly Leu Gly Gly
            420                 425                 430

Gln Gly Ala Gly Ala Ala Ala Ala Ala Ala Ala Gly Gly Ala Gly Gln
        435                 440                 445

Gly Gly Tyr Gly Gly Leu Gly Asn Gln Gly Ala Gly Arg Gly Gly Gln
    450                 455                 460

Gly Ala Ala Ala Ala Ala Gly Gly Ala Gly Gln Gly Gly Tyr Gly Gly
465                 470                 475                 480

Leu Gly Ser Gln Gly Ala Gly Arg Gly Gly Gln Gly Ala Gly Ala Ala
                485                 490                 495

Ala Ala Ala Ala Val Gly Ala Gly Gln Glu Gly Ile Arg Gly Gln Gly
            500                 505                 510

Ala Gly Gln Gly Gly Tyr Gly Gly Leu Gly Ser Gln Gly Ser Gly Arg
        515                 520                 525

Gly Gly Leu Gly Gly Gln Gly Ala Gly Ala Ala Ala Ala Ala Ala Gly
    530                 535                 540

Gly Ala Gly Gln Gly Gly Leu Gly Gly Gln Gly Ala Gly Gln Gly Ala
545                 550                 555                 560

Gly Ala Ala Ala Ala Ala Ala Gly Gly Val Arg Gln Gly Gly Tyr Gly
                565                 570                 575

Gly Leu Gly Ser Gln Gly Ala Gly Arg Gly Gly Gln Gly Ala Gly Ala
            580                 585                 590

Ala Ala Ala Ala Ala Gly Gly Ala Gly Gln Gly Gly Tyr Gly Gly Leu
        595                 600                 605

Gly Gly Gln Gly Val Gly Arg Gly Gly Leu Gly Gly Gln Gly Ala Gly
    610                 615                 620

Ala Ala Ala Ala Gly Gly Ala Gly Gln Gly Gly Tyr Gly Gly Val Gly
625                 630                 635                 640

Ser Gly Ala Ser Ala Ala Ser Ala Ala Ala Ser Arg Leu Ser Ser Pro
                645                 650                 655

Gln Ala Ser Ser Arg Val Ser Ser Ala Val Ser Asn Leu Val Ala Ser
            660                 665                 670

Gly Pro Thr Asn Ser Ala Ala Leu Ser Ser Thr Ile Ser Asn Val Val
        675                 680                 685

Ser Gln Ile Gly Ala Ser Ile Leu Val Phe Leu Asp Val Met Ser Ser
    690                 695                 700

Phe Lys Leu Phe Ser Arg Leu Phe Leu Leu Leu Ser Arg Ser
705                 710                 715

(2) INFORMATION FOR SEQ ID NO:3:

     (i) SEQUENCE CHARACTERISTICS:
          (A) LENGTH: 1995 base pairs
          (B) TYPE: nucleic acid
          (C) STRANDEDNESS: single
          (D) TOPOLOGY: linear

    (ii) MOLECULE TYPE: cDNA

   (iii) HYPOTHETICAL: NO

   (vii) IMMEDIATE SOURCE:
          (B) CLONE: p6B

(ix) FEATURE:
(A) NAME/KEY: CDS
(B) LOCATION: 1..1785

(xi) SEQUENCE DESCRIPTION: SEQ ID NO:3:

CCT GGA GGA TAT GGA CCA GGA CAA CAA GGC CCA GGA GGA TAT GGC CCT 48
Pro Gly Gly Tyr Gly Pro Gly Gln Gln Gly Pro Gly Gly Tyr Gly Pro
1               5                   10                  15

GGA CAA CAA GGA CCA TCT GGA CCT GGC AGT GCC GCT GCA GCA GCA GCA 96
Gly Gln Gln Gly Pro Ser Gly Pro Gly Ser Ala Ala Ala Ala Ala Ala
            20                  25                  30

GCC GCC GCA GCA GGA CCT GGA GGA TAT GGC CCT GGA CAA CAA GGA CCC 144
Ala Ala Ala Ala Gly Pro Gly Gly Tyr Gly Pro Gly Gln Gln Gly Pro
        35                  40                  45

GGA GGA TAT GGA CCA GGA CAA CAA GGA CCC GGA AGA TAT GGA CCA GGA 192
Gly Gly Tyr Gly Pro Gly Gln Gln Gly Pro Gly Arg Tyr Gly Pro Gly
    50                  55                  60

CAA CAA GGA CCA TCT GGA CCT GGC AGT GCC GCT GCA GCC GCA GCA GGA 240
Gln Gln Gly Pro Ser Gly Pro Gly Ser Ala Ala Ala Ala Ala Ala Gly
65                  70                  75                  80

TCT GGA CAA CAA GGC CCA GGA GGA TAT GGA CCA CGT CAA CAA GGT CCA 288
Ser Gly Gln Gln Gly Pro Gly Gly Tyr Gly Pro Arg Gln Gln Gly Pro
                85                  90                  95

[?]GA GGT TAT GGA CAA GGA CAA CAA GGA CCA TCT GGA CCA GGC AGT GCA 336
[?]ly Gly Tyr Gly Gln Gly Gln Gln Gly Pro Ser Gly Pro Gly Ser Ala
100 105 110

GCC GCA GCC TCA GCC GCA GCC TCA GCA GAA TCT GGA CAA CAA GGC CCA 384
Ala Ala Ala Ser Ala Ala Ala Ser Ala Glu Ser Gly Gln Gln Gly Pro
115 120 125

GGA GGT TAT GGA CCA GGT CAA CAA GGC CCA GGA GGT TAT GGA CCA GGT 432
Gly Gly Tyr Gly Pro Gly Gln Gln Gly Pro Gly Gly Tyr Gly Pro Gly
130 135 140

CAA CAA GGT CCT GGA GGA TAT GGA CCA GGA CAA CAA GGA CCA TCT GGA 480
Gln Gln Gly Pro Gly Gly Tyr Gly Pro Gly Gln Gln Gly Pro Ser Gly
145 150 155 160

CCA GGT AGT GCC GCT GCA GCA GCC GCC GCC GCA TCA GGA CCT GGA CAA 528
Pro Gly Ser Ala Ala Ala Ala Ala Ala Ala Ala Ser Gly Pro Gly Gln
165 170 175

CAA GGA CCA GGA GGA TAT GGA CCA GGT CAA CAA GGT CCT GGA GGA TAT 576
Gln Gly Pro Gly Gly Tyr Gly Pro Gly Gln Gln Gly Pro Gly Gly Tyr
180 185 190

GGA CCA GGA CAA CAA GGA CCA TCT GGA CCA GGT AGT GCC GCT GCA GCC 624
Gly Pro Gly Gln Gln Gly Pro Ser Gly Pro Gly Ser Ala Ala Ala Ala
195 200 205

GCC GCC GCC GCA TCA GGA CCT GGA CAA CAA GGA CCA GGA GGA TAT GGA 672
Ala Ala Ala Ala Ser Gly Pro Gly Gln Gln Gly Pro Gly Gly Tyr Gly
210 215 220

CCA GGT CAA CAA GGT CCA GGA GGT TAT GGA CCA GGA CAA CAA GGA CTA 720
Pro Gly Gln Gln Gly Pro Gly Gly Tyr Gly Pro Gly Gln Gln Gly Leu
225 230 235 240

[?]CT GGA CCA GGC AGT GCA GCT GCA GCA GCC GCA GCA GGA CCT GGA CAA 768
[?]er Gly Pro Gly Ser Ala Ala Ala Ala Ala Ala Ala Gly Pro Gly Gln
245 250 255

CAA GGA CCC GGA GGA TAT GGA CCA GGA CAA CAA GGA CCA TCT GGA CCC 816
Gln Gly Pro Gly Gly Tyr Gly Pro Gly Gln Gln Gly Pro Ser Gly Pro
260 265 270

GGT AGT GCC GCT GCA GCA GCA GCC GCC GCA GCA GGA CCT GGA GGA TAT 864
Gly Ser Ala Ala Ala Ala Ala Ala Ala Ala Ala Gly Pro Gly Gly Tyr
275 280 285

GGC CCT GGA CAA CAA GGA CCC GGA GGA TAT GGA CCA GGA CAA CAA GGA 912
Gly Pro Gly Gln Gln Gly Pro Gly Gly Tyr Gly Pro Gly Gln Gln Gly
290 295 300

CCA TCT GGA GCA GGC AGT GCA GCA GCA GCA GCC GCA GCA GGA CCT GGA 960
Pro Ser Gly Ala Gly Ser Ala Ala Ala Ala Ala Ala Ala Gly Pro Gly
305 310 315 320

[?]AA CAA GGA TTA GGA GGT TAT GGA CCA GGA CAA CAA GGT CCA GGA GGA 1008
Gln Gln Gly Leu Gly Gly Tyr Gly Pro Gly Gln Gln Gly Pro Gly Gly
325 330 335

TAT GGA CCA GGA CAA CAA GGT CCA GGA GGA TAT GGA CCA GGT AGT GCA 1056
Tyr Gly Pro Gly Gln Gln Gly Pro Gly Gly Tyr Gly Pro Gly Ser Ala
340 345 350

TCT GCA GCA GCA GCC GCA GCA GGA CCT GGA CAA CAA GGA CCA GGA GGA 1104
Ser Ala Ala Ala Ala Ala Ala Gly Pro Gly Gln Gln Gly Pro Gly Gly
355 360 365

TAT GGA CCT GGA CAA CAA GGA CCA TCT GGA CCA GGC AGT GCA TCT GCA 1152
Tyr Gly Pro Gly Gln Gln Gly Pro Ser Gly Pro Gly Ser Ala Ser Ala
370 375 380

GCA GCA GCC GCA GCC GCA GCA GGA CCA GGA GGA TAT GGA CCA GGA CAA 1200
Ala Ala Ala Ala Ala Ala Ala Gly Pro Gly Gly Tyr Gly Pro Gly Gln
385 390 395 400

CAA GGT CCA GGA GGA TAT GCA CCA GGA CAA CAA GGA CCA TCT GGA CCA 1248
Gln Gly Pro Gly Gly Tyr Ala Pro Gly Gln Gln Gly Pro Ser Gly Pro
405 410 415

GGC AGT GCA TCT GCA GCA GCA GCC GCA GCC GCA GCA GGA CCA GGA GGA 1296
Gly Ser Ala Ser Ala Ala Ala Ala Ala Ala Ala Ala Gly Pro Gly Gly
420 425 430

TAT GGA CCA GGA CAA CAA GGT CCA GGA GGA TAT GCA CCA GGA CAA CAA 1344
Tyr Gly Pro Gly Gln Gln Gly Pro Gly Gly Tyr Ala Pro Gly Gln Gln
435 440 445

GGA CCA TCT GGA CCA GGC AGT GCA GCA GCA GCA GCA GCT GCC AGT GCA 1392
Gly Pro Ser Gly Pro Gly Ser Ala Ala Ala Ala Ala Ala Ala Ser Ala
450 455 460

GGA CCT GGT GGA TAT GGA CCA GCG CAA CAG GGA CCA TCT GGT CCT GGA 1440
Gly Pro Gly Gly Tyr Gly Pro Ala Gln Gln Gly Pro Ser Gly Pro Gly
465 470 475 480

ATC GCA GCT TCA GCT GCT TCA GCA GGA CCT GGA GGT TAT GGA CCA GCA 1488
Ile Ala Ala Ser Ala Ala Ser Ala Gly Pro Gly Gly Tyr Gly Pro Ala
485 490 495

CAA CAA GGA CCA GCT GGA TAT GGG CCT GGA AGC GCA GTA GCA GCC TCT 1536
Gln Gln Gly Pro Ala Gly Tyr Gly Pro Gly Ser Ala Val Ala Ala Ser
500 505 510

GCC GGT GCA GGA TCT GCA GGT TAT GGG CCA GGT TCT CAA GCT TCC GCT 1584
Ala Gly Ala Gly Ser Ala Gly Tyr Gly Pro Gly Ser Gln Ala Ser Ala
515 520 525

GCA GCT TCT CGT CTG GCT TCT CCA GAT TCA GGC GCT AGA GTT GCA TCA 1632
Ala Ala Ser Arg Leu Ala Ser Pro Asp Ser Gly Ala Arg Val Ala Ser
530 535 540

GCT GTT TCT AAC TTG GTA TCC AGT GGC CCA ACT AGC TCT GCT GCC TTA 1680
Ala Val Ser Asn Leu Val Ser Ser Gly Pro Thr Ser Ser Ala Ala Leu
545 550 555 560

TCA AGT GTT ATC AGT AAC GCT GTG TCT CAA ATT GGC GCA AGT AAT CCT 1728
Ser Ser Val Ile Ser Asn Ala Val Ser Gln Ile Gly Ala Ser Asn Pro
565 570 575

GGT CTC TCT GGT TGC GAT GTC CTC ATT CAA GCT CTC TGG AAA TCG TTT 1776
Gly Leu Ser Gly Cys Asp Val Leu Ile Gln Ala Leu Trp Lys Ser Phe
580 585 590

CTG CTT GTG TAA CCA TCC TTT CTT CAT CCA GCA TTG GTC AAG TTA ATT 1824
Leu Leu Val
595

ATG GAG CGG CTT CTC AGT TCG CCC AAG TTG TCG GCC AAT CTG TTT TGA 1872

GTG CAT TTT AAT TGA AAA ATT TAT TAA AAT ATG CAT GGA TTT TCT AGC 1920

CTG GGC AAC TAA TTG CTC GTA CTA TGT AAT TTT TTT TTA AAT AAA TTC 1968

TTT GCA ACT TCT AAA AAA AAA AAA AAA 1995

(2) INFORMATION FOR SEQ ID NO:4:

(i) SEQUENCE CHARACTERISTICS:
(A) LENGTH: 596 amino acids
(B) TYPE: amino acid
(D) TOPOLOGY: linear

(ii) MOLECULE TYPE: protein

(xi) SEQUENCE DESCRIPTION: SEQ ID NO:4:

Pro Gly Gly Tyr Gly Pro Gly Gln Gln Gly Pro Gly Gly Tyr Gly Pro
1               5                   10                  15

Gly Gln Gln Gly Pro Ser Gly Pro Gly Ser Ala Ala Ala Ala Ala Ala
            20                  25                  30

Ala Ala Ala Ala Gly Pro Gly Gly Tyr Gly Pro Gly Gln Gln Gly Pro
        35                  40                  45

Gly Gly Tyr Gly Pro Gly Gln Gln Gly Pro Gly Arg Tyr Gly Pro Gly
    50                  55                  60

[?]ln Gln Gly Pro Ser Gly Pro Gly Ser Ala Ala Ala Ala Ala Ala Gly
[?]5 70 75 80

Ser Gly Gln Gln Gly Pro Gly Gly Tyr Gly Pro Arg Gln Gln Gly Pro
85 90 95

Gly Gly Tyr Gly Gln Gly Gln Gln Gly Pro Ser Gly Pro Gly Ser Ala
100 105 110

Ala Ala Ala Ser Ala Ala Ala Ser Ala Glu Ser Gly Gln Gln Gly Pro
115 120 125

Gly Gly Tyr Gly Pro Gly Gln Gln Gly Pro Gly Gly Tyr Gly Pro Gly
130 135 140

Gln Gln Gly Pro Gly Gly Tyr Gly Pro Gly Gln Gln Gly Pro Ser Gly
145 150 155 160

Pro Gly Ser Ala Ala Ala Ala Ala Ala Ala Ala Ser Gly Pro Gly Gln
165 170 175

Gln Gly Pro Gly Gly Tyr Gly Pro Gly Gln Gln Gly Pro Gly Gly Tyr
180 185 190

Gly Pro Gly Gln Gln Gly Pro Ser Gly Pro Gly Ser Ala Ala Ala Ala
        195                 200                 205

Ala Ala Ala Ala Ser Gly Pro Gly Gln Gln Gly Pro Gly Gly Tyr Gly
    210                 215                 220

Pro Gly Gln Gln Gly Pro Gly Gly Tyr Gly Pro Gly Gln Gln Gly Leu
225                 230                 235                 240

Ser Gly Pro Gly Ser Ala Ala Ala Ala Ala Ala Ala Gly Pro Gly Gln
                245                 250                 255

[?]ln Gly Pro Gly Gly Tyr Gly Pro Gly Gln Gln Gly Pro Ser Gly Pro
            260                 265                 270

Gly Ser Ala Ala Ala Ala Ala Ala Ala Ala Ala Gly Pro Gly Gly Tyr
        275                 280                 285

Gly Pro Gly Gln Gln Gly Pro Gly Gly Tyr Gly Pro Gly Gln Gln Gly
    290                 295                 300

Pro Ser Gly Ala Gly Ser Ala Ala Ala Ala Ala Ala Ala Gly Pro Gly
305                 310                 315                 320

Gln Gln Gly Leu Gly Gly Tyr Gly Pro Gly Gln Gln Gly Pro Gly Gly
                325                 330                 335

Tyr Gly Pro Gly Gln Gln Gly Pro Gly Gly Tyr Gly Pro Gly Ser Ala
            340                 345                 350

Ser Ala Ala Ala Ala Ala Ala Gly Pro Gly Gln Gln Gly Pro Gly Gly
        355                 360                 365

Tyr Gly Pro Gly Gln Gln Gly Pro Ser Gly Pro Gly Ser Ala Ser Ala
    370                 375                 380

Ala Ala Ala Ala Ala Ala Ala Gly Pro Gly Gly Tyr Gly Pro Gly Gln
385                 390                 395                 400

Gln Gly Pro Gly Gly Tyr Ala Pro Gly Gln Gln Gly Pro Ser Gly Pro
                405                 410                 415

Gly Ser Ala Ser Ala Ala Ala Ala Ala Ala Ala Ala Gly Pro Gly Gly
            420                 425                 430

Tyr Gly Pro Gly Gln Gln Gly Pro Gly Gly Tyr Ala Pro Gly Gln Gln
        435                 440                 445

Gly Pro Ser Gly Pro Gly Ser Ala Ala Ala Ala Ala Ala Ala Ser Ala
    450                 455                 460

Gly Pro Gly Gly Tyr Gly Pro Ala Gln Gln Gly Pro Ser Gly Pro Gly
465                 470                 475                 480

Ile Ala Ala Ser Ala Ala Ser Ala Gly Pro Gly Gly Tyr Gly Pro Ala
                485                 490                 495

Gln Gln Gly Pro Ala Gly Tyr Gly Pro Gly Ser Ala Val Ala Ala Ser
            500                 505                 510

Ala Gly Ala Gly Ser Ala Gly Tyr Gly Pro Gly Ser Gln Ala Ser Ala
        515                 520                 525

Ala Ala Ser Arg Leu Ala Ser Pro Asp Ser Gly Ala Arg Val Ala Ser
    530                 535                 540

[?]la Val Ser Asn Leu Val Ser Ser Gly Pro Thr Ser Ser Ala Ala Leu
[?]5                 550                 555                 560

Ser Ser Val Ile Ser Asn Ala Val Ser Gln Ile Gly Ala Ser Asn Pro
                565                 570                 575

Gly Leu Ser Gly Cys Asp Val Leu Ile Gln Ala Leu Trp Lys Ser Phe
            580                 585                 590

Leu Leu Val
        595

## Description

### FIELD OF THE INVENTION

The invention relates to a DNA molecule which codes for a silk protein, a vector or plasmid which contains said DNA molecule, a process for preparing recombinant spider silk proteins and to spider silk proteins and fragments and variants thereof.

### BACKGROUND OF THE INVENTION

Major ampullate (dragline) silk of orb web spiders possesses unique physical properties, combining high tensile strength and substantial elasticity, Denny, M.W. J. Exp. Biol., 65, 483-506 (1976); Lucas, F. Discovery, 25, 20-26 (1964). Previous investigations suggest that spider silk is composed of a single large protein, primarily containing pseudo-crystalline regions of stack β-pleated sheet alternating with amorphous domains, Warwicker, J.O., J.Mol.Biol., 2, 350-362 (1960); Lucase, F. et al, J.Text Inst., 46, T440-T452 (1985); Hepburn, H.R. et al., Insect Biochem., 9, 69-71 (1979). The molecular basis for spider silk elasticity is presently unknown, although it has been suggested that an entropy driven process like that found in rubber is involved, Gosline, J.M., et al., Nature, 309, 551-552 (1984). It has also been speculated that the amorphous regions contribute substantially to the elastic properties of the fiber, Hepburn, H.R., et al., Insect Biochem., 9, 69-77 (1979).

### SUMMARY OF THE INVENTION

It is an object of the present invention to provide the DNA and means for the production of silk proteins and variants thereof by recombiant DNA techniques. It is also an object of the invention to provide fragments and variants of recombinant silk proteins. These objects are achieved by the invention as defined in claims 1 to 53. Preferred embodiments are described in the dependent claims.

Specifically, the inventors have discovered that spider silk protein from Nephila clavipes naturally occurs as a mixture of at least two spider silk proteins. The inventors have cloned portions of the genes for both of these proteins and have named these proteins Silk Protein 1 and Silk Protein 2. Because these two genes have been independently cloned, these two proteins can be independently prepared by recombinant techniques. The silk proteins can then be purified, i.e., separated from contaminating materials in the expression system, to produce purified or homogeneous Silk protein 1 or purified or homogeneous Silk Protein 2. The spider Silk Protein 1 is therefore free from spider Silk Protein 2 and the spider Silk Protein 2 is free from spider Silk Protein 1. These proteins can be used in a pure form or they can be mixed with each other in order to approximate the properties of natural spider silk. The silk Protein 1 and silk Protein 2 can be mixed in an amount of 100:1 to 1:100, preferably 10:1 to 1:2, more preferably 5:1 to 1:1.

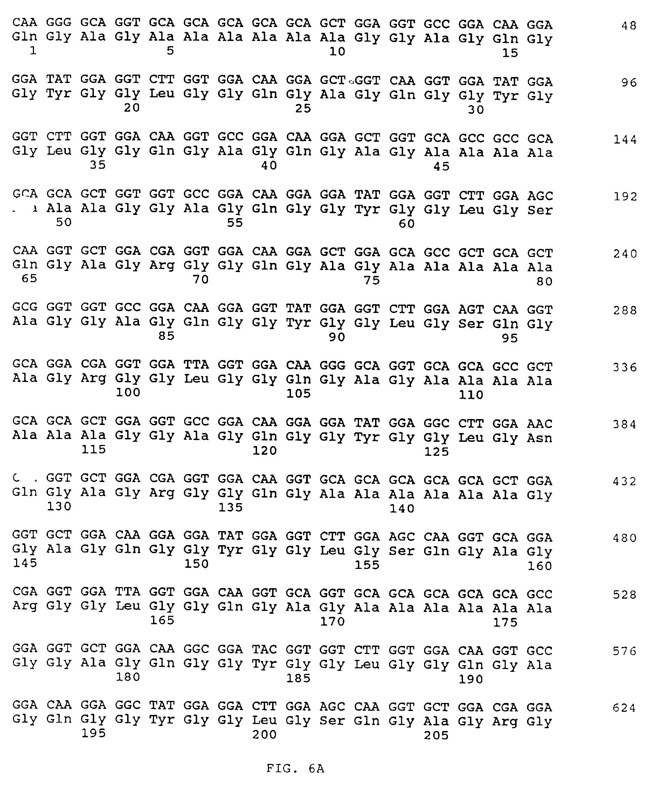

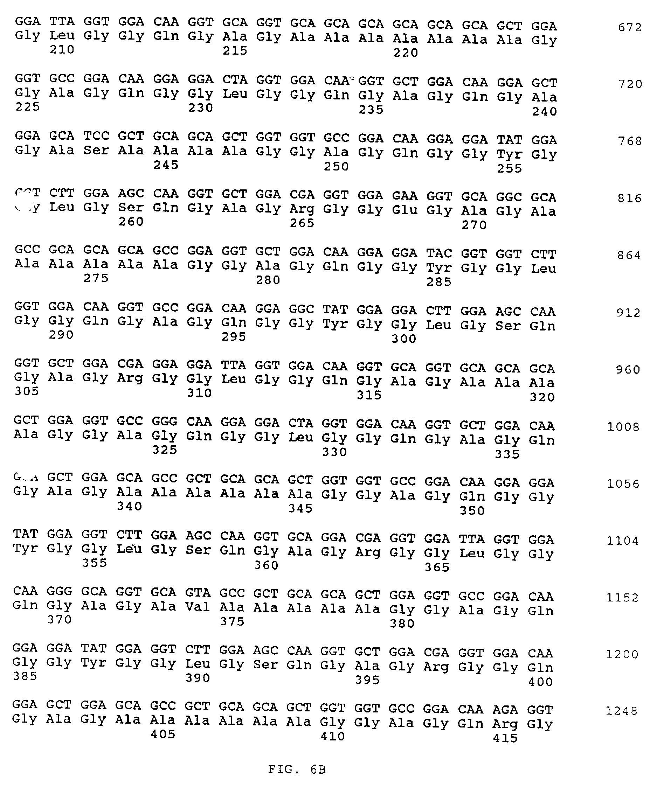

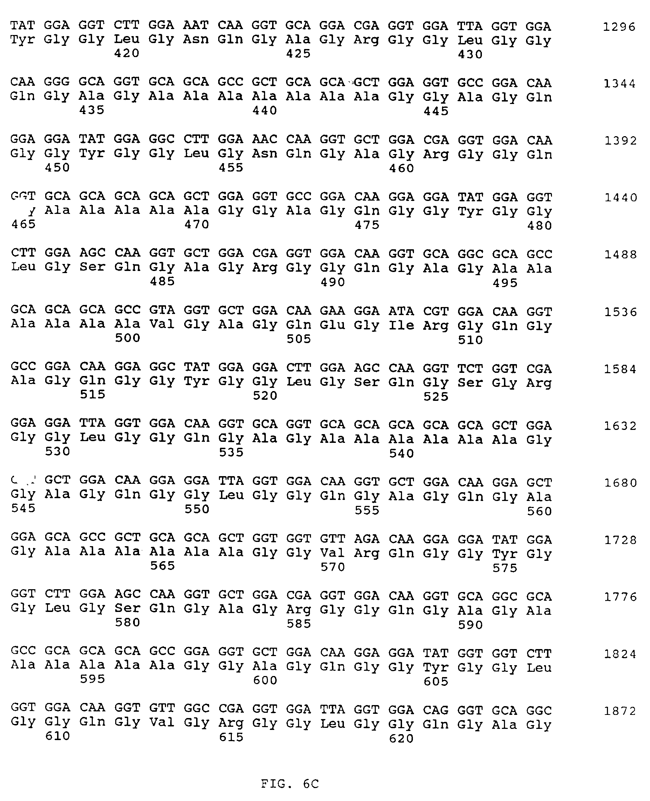

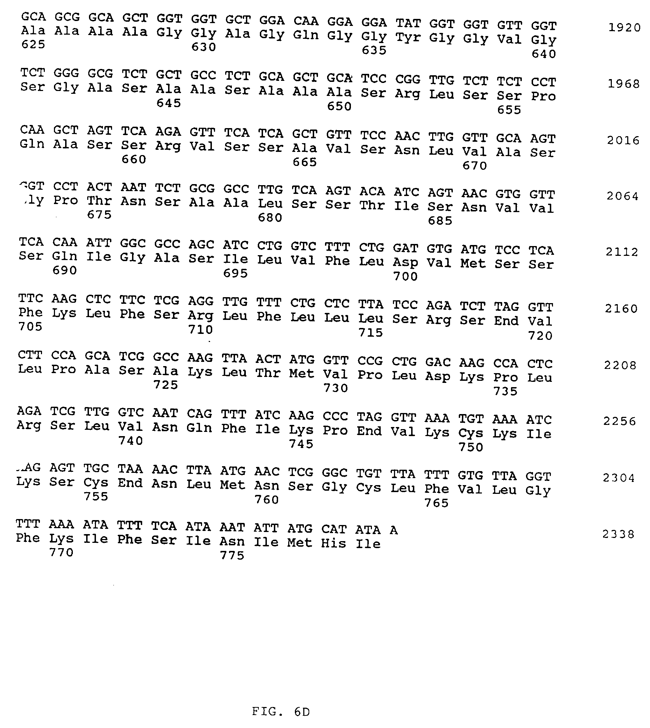

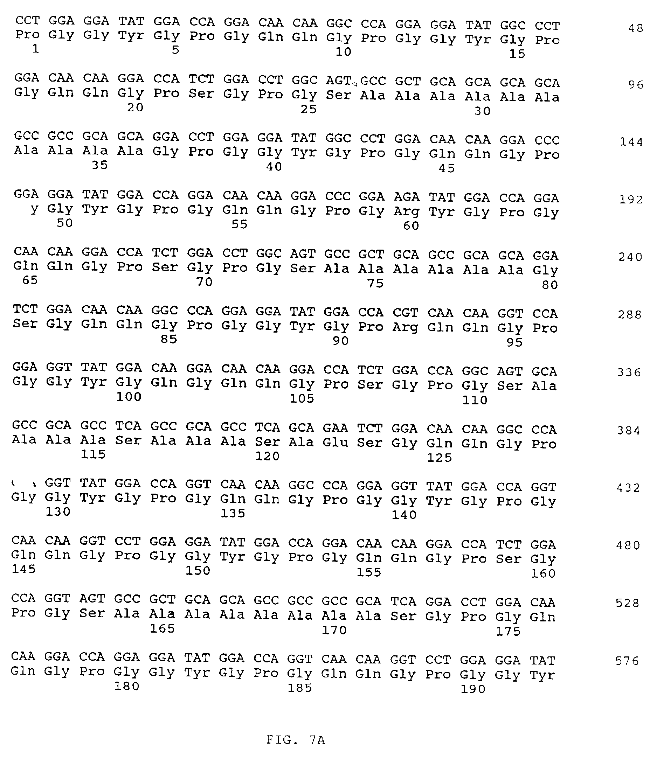

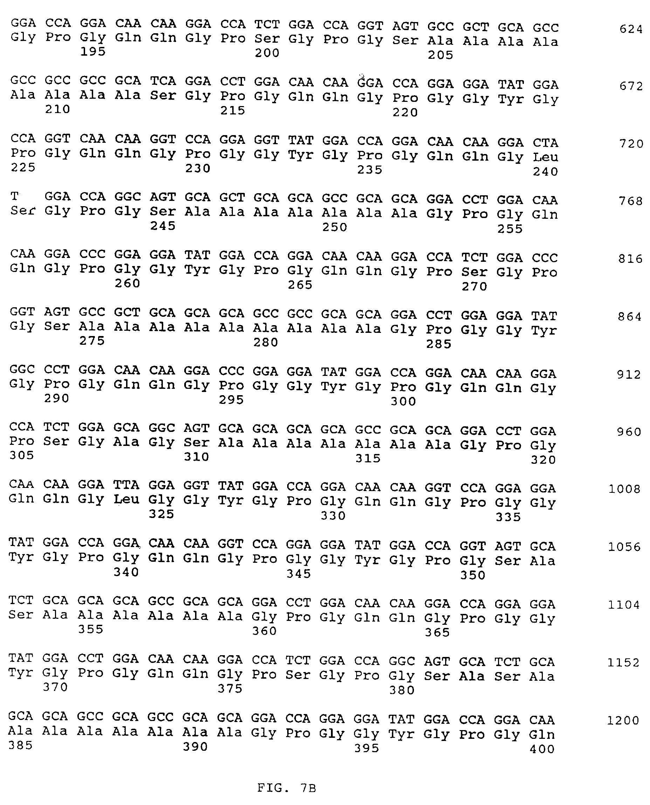

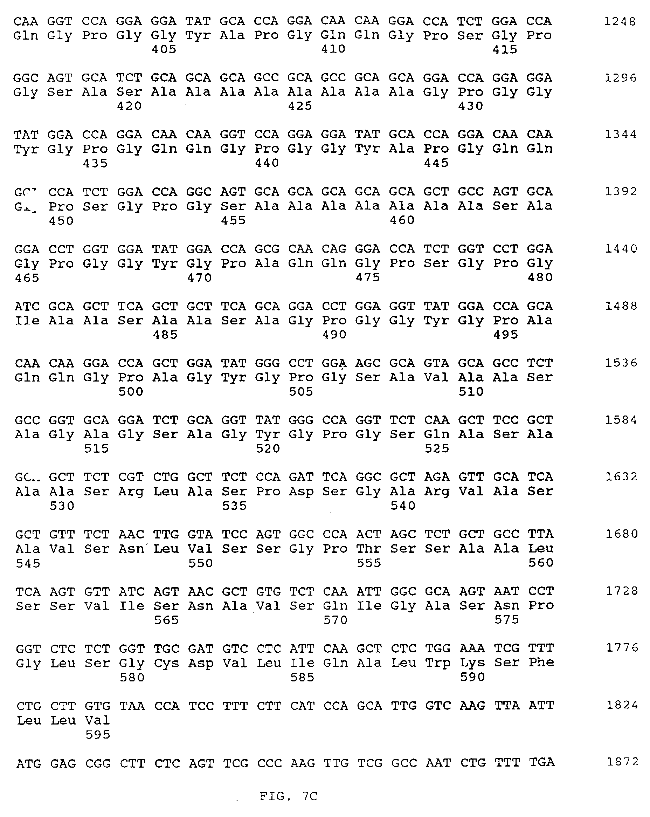

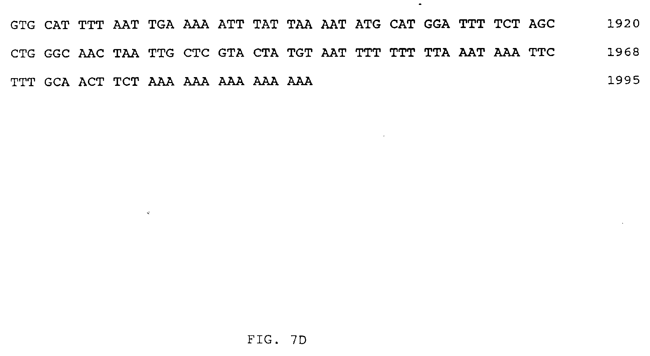

The isolated cDNA of the invention preferably codes for spider silk protein containing the sequence shown in Figure 6 or Figure 7 or a fragment or variant thereof.

The amino acid composition of the fragment or variant thereof may match that of the native spider silk protein. The structure, via FTIR, may show predominantly a β-sheet structure. The fragment of variant, like the native protein, should be soluble only in highly chaotropic solvents such LiSCN, Li perchlorate or formic acid.

The spider silk protein can be characterized by repeating α and β regions and optional variable regions. The full cDNA spider Silk Protein 1 and spider Silk Protein 2 have not been cloned or sequenced. However, it can be expected that spider Silk Protein 1 and spider Silk Protein 2 each may have a molecular weight less than 300,000 daltons, probably greater than 100,000 but less than 300,000 daltons, preferably 120,000 to 300,000 daltons. Spider Silk Protein 1 and spider Silk Protein 2 may each have 900 to 2700 amino acids with 25 to 100, preferably 30 to 90 repeats. For example, the spider silk protein may be represented by the formula [(α)(β)]ₚ
wherein α is an amorphous region which can form an α-helix when stretched, β is a region which can form β-sheets (when in a folded conformation) and p is an integer of 1 to 100, preferably 15 to 50, more preferably 18 to 22 for fragments or p is an integer of 25 to 100, preferably 30 to 90 for the full length spider silk protein. The sequence may also contain optional variable regions interspersed between the α and/or β regions.

A useful protein or fragment should be (1) insoluble inside the cell in which it is expressed or (2) capable of being formed into an insoluble fiber under normal conditions by which fibers are made. Preferably, the protein is insoluble under conditions (1) and (2). Specifically, the protein or fragment should be insoluble in a solvent such as water, alcohol (methanol, ethanol, etc.), acetone and/or organic acids, etc. The protein or fragment should be capable of being formed into a fiber having high tensile strength, e.g., a tensile strength of 0.5x to 2x wherein x is the tensile strength of a fiber formed from the corresponding natural silk or the whole protein. The protein or fragment should also be capable of being formed into a fiber possessing an elasticity of at least 15%, more preferably about 25%.

Variants of the spider silk protein may be formed into a fiber having a tensile strength and/or elasticity which is greater than that of the natural spider silk or natural protein. The elasticity could possible be increased up to 100%. The variants may also possess the properties of the above described fragments.

The fragment or variant may have substantially the same characteristics as the natural spider silk. The natural protein is particularly insoluble when in the fiber form and is resistant to degradation by most enzymes.

In the present invention, the isolated cDNA may code for spider silk proteins such as Nephila clavipes major ampullate (dragline) silk protein, Nephila clavipes minor ampullate silk protein, Nephila clavipes cocoon silk protein, Areneus gemmoides major ampullate silk protein, and Areneus gemmoides cocoon silk protein.

The invention is further directed to a replicable vector containing cDNA which codes for spider silk protein and which is capable of expressing spider silk protein.

The invention further relates to a transformed cell or microorganism containing cDNA or a vector which codes for spider silk protein or a fragment or variant thereof and which is capable of expressing spider silk protein.

The present invention is also directed to a new silk protein and a method for producing the protein which comprises culturing the transformed cell or microorganism described above under conditions which allow expression of the silk protein, optionally recovering the thus expressed silk protein and optionally purifying the recovered silk protein. The silk protein produced in this manner may be different from natural spider silk protein. The silk protein produced by recombinant techniques may also contain some small amounts of contaminating materials from the microorganism, cells and/or fermentation system in which it was produced.

### BRIEF DESCRIPTION OF THE DRAWINGS

The present invention will become more fully understood from the detailed description given hereinbelow, and the accompanying drawings which are given by way of illustration only, and thus are not limitative of the present invention, and wherein;

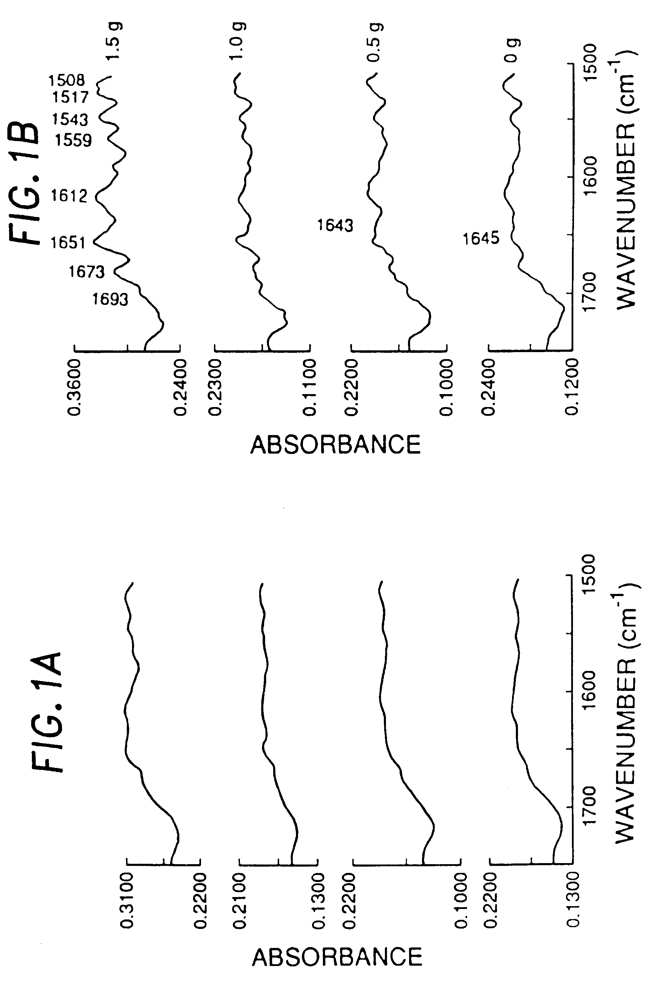

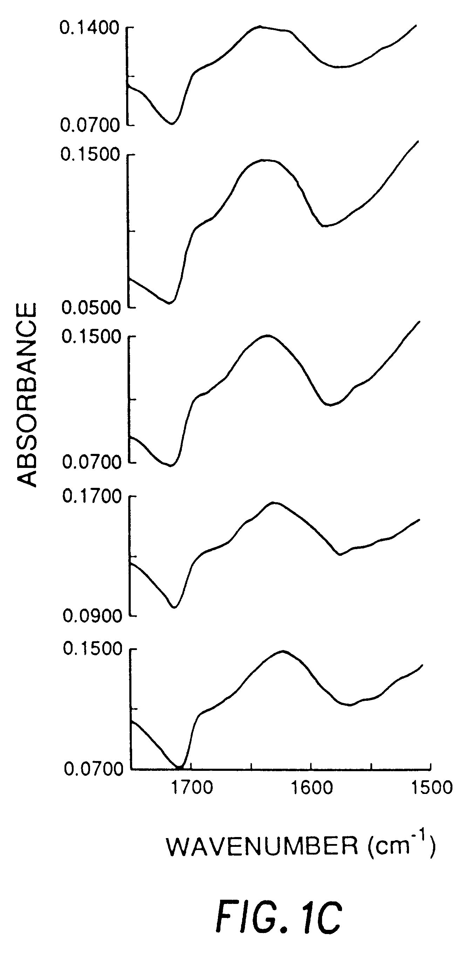

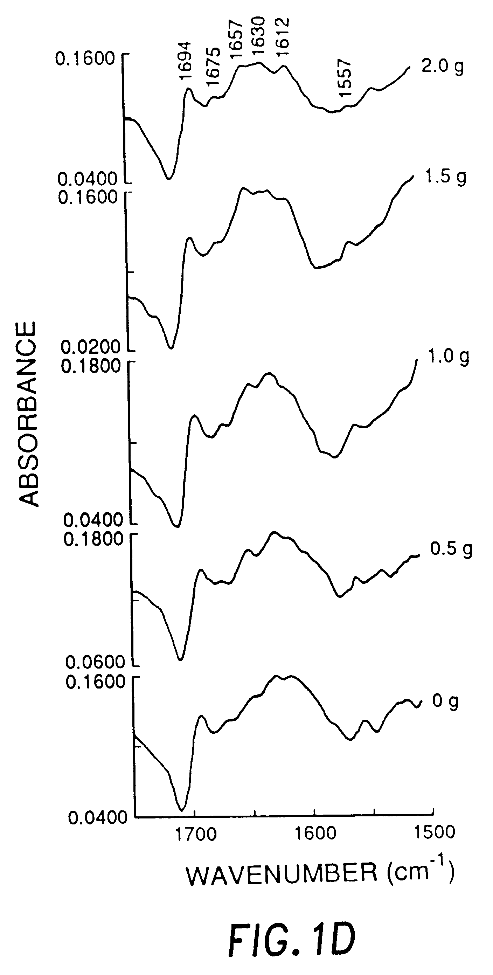

FIGURE 1 shows the FTIR Spectra of dragline silk fiber from the major ampullate gland of Nephila clavipes as a function of applied force with fiber axis perpendicular (C and D) and parallel (A and B) to the polarized radiation; A) and C): original spectra; B) and D): partially deconvoluted spectra of A) and C), respectively;

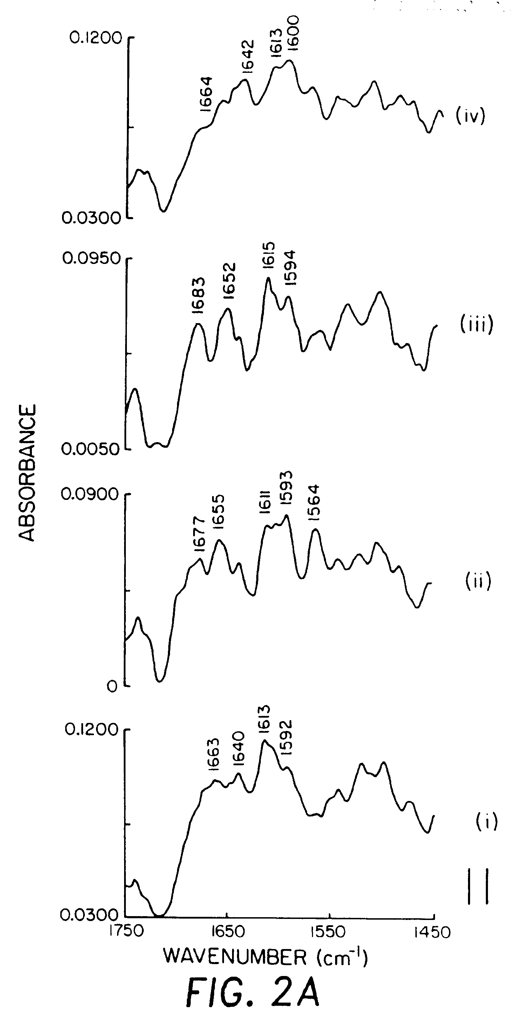

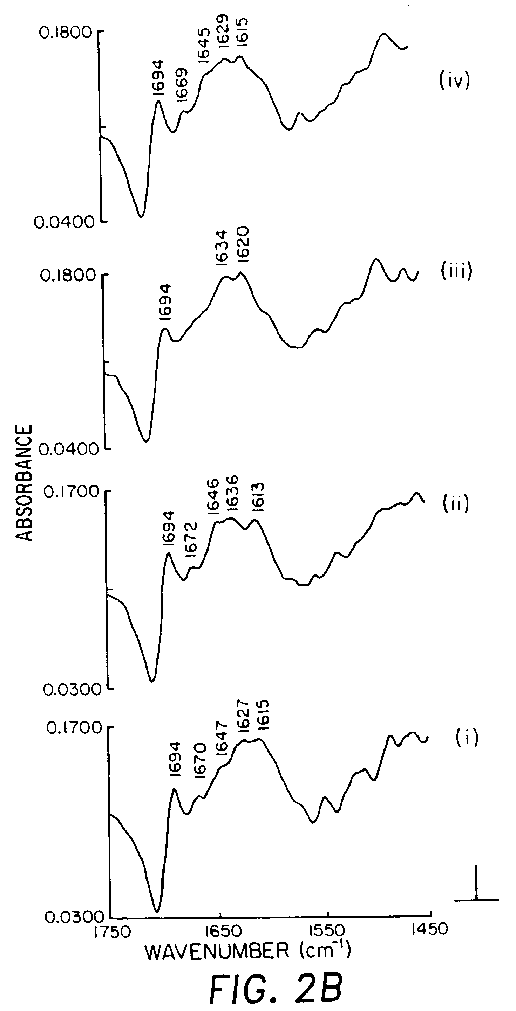

FIGURE 2 shows the FTIR Spectra of dragline silk fiber from the major ampullate gland of Nephila clavipes with fiber axis parallel (A) and perpendicular (B) to the polarized radiation; (i): 0 gram axial tension; (ii): 2.0 gram axial tension; (iii): 29 min after release of the axial tension; (iv): 12 hrs after release of the axial tension;

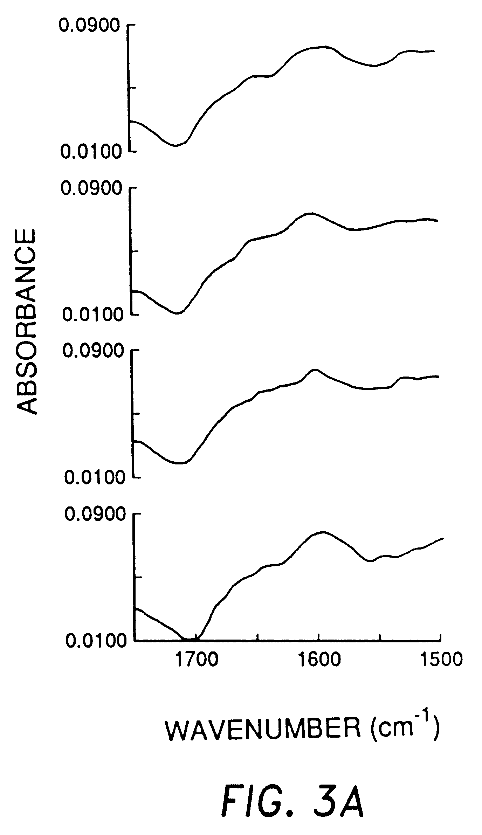

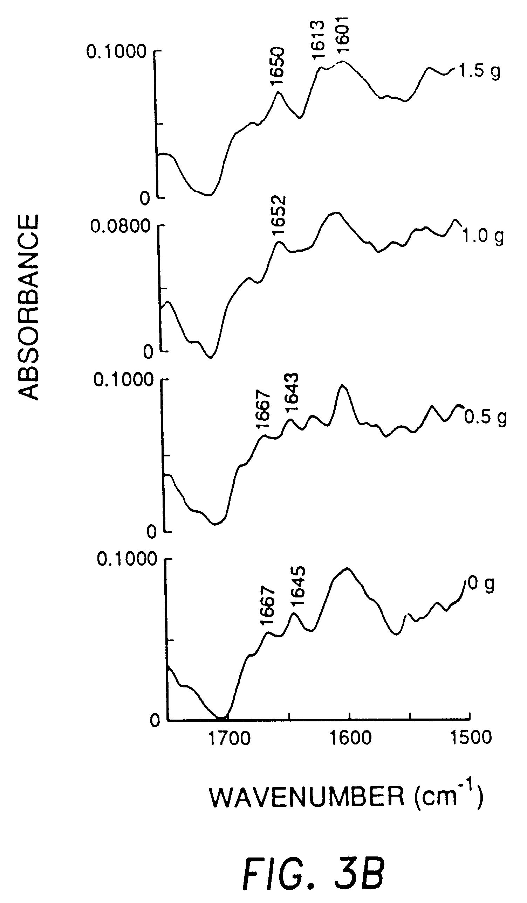

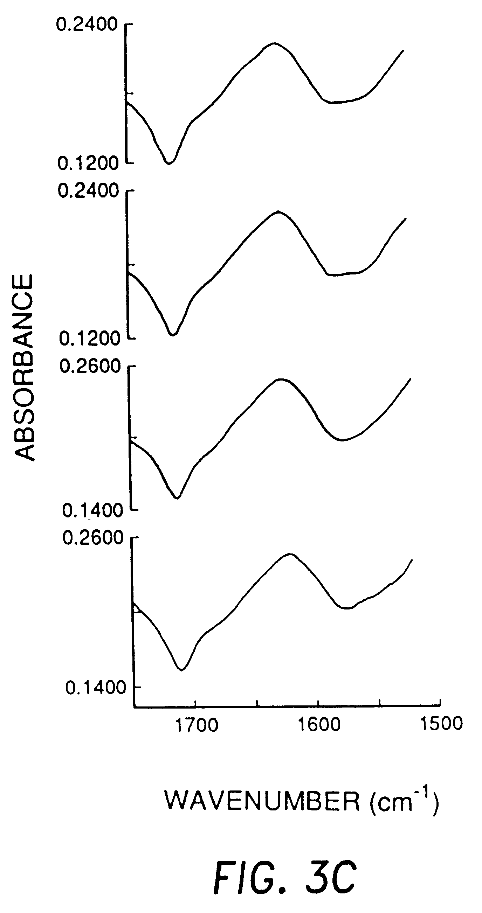

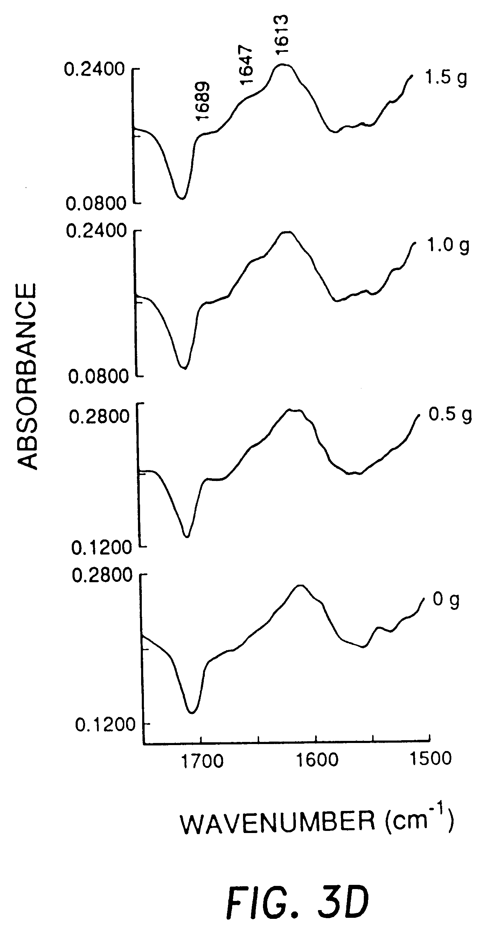

FIGURE 3 shows the FTIR Spectra of dragline silk from the major ampullate gland of Araneus gemmoides as a function of applied force with fiber axis perpendicular (C and D) and parallel (A and B) to the polarized radiation; A) and C): original spectra; E) and D): partially deconvoluted spectra of A) and C), respectively;

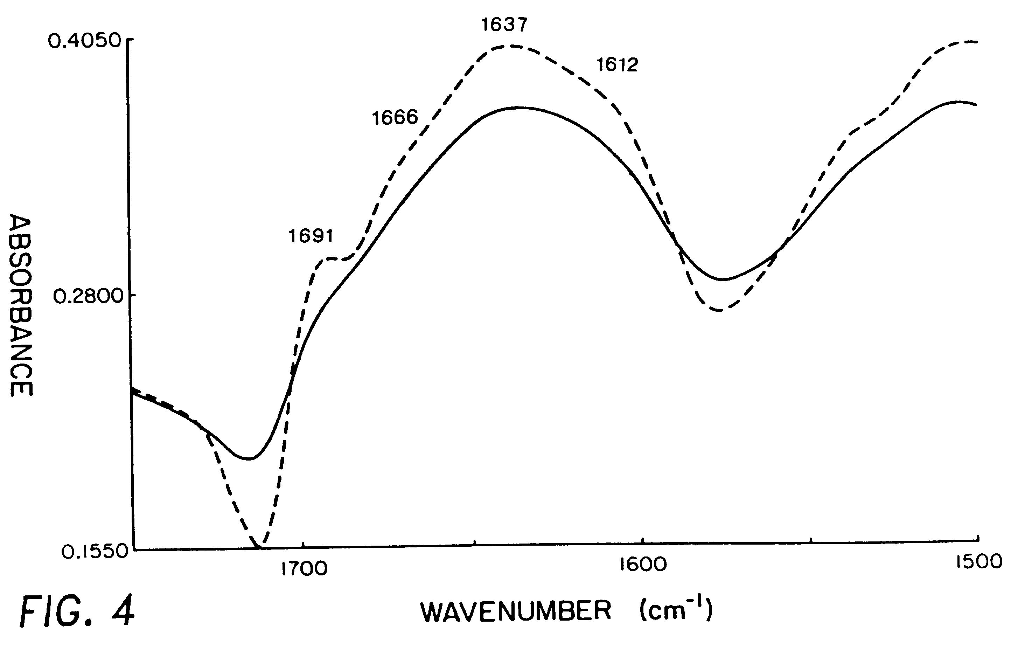

FIGURE 4 shows the FTIR Spectra of 3 strands of dragline silk from the major ampullate gland of Nephila clavipes being oriented randomly on the sample plate and detected without polarizer; Solid line: original spectrum; Dashed line: partially deconvoluted spectrum;

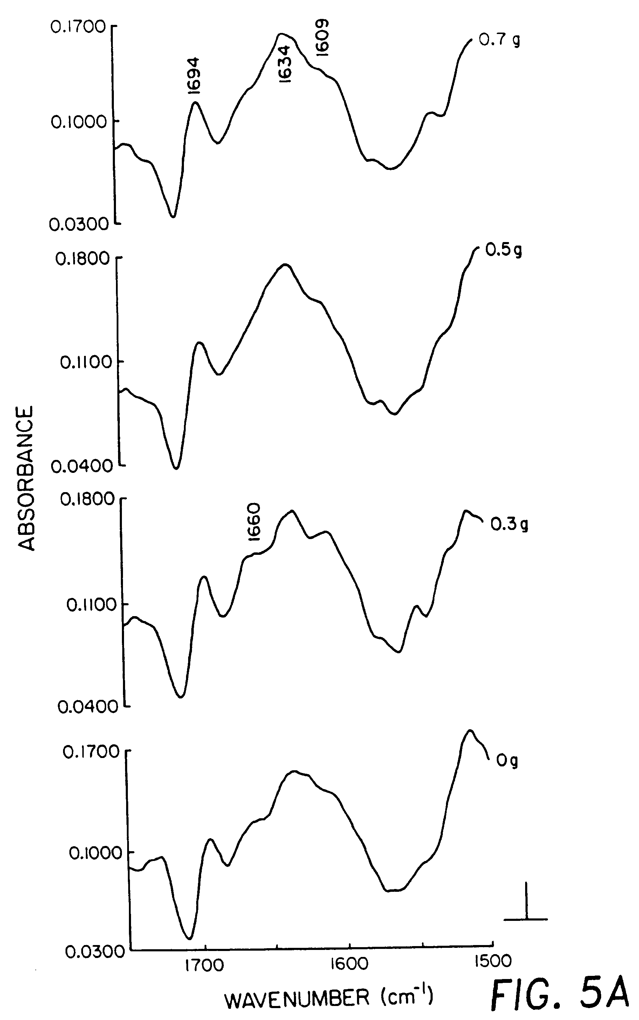

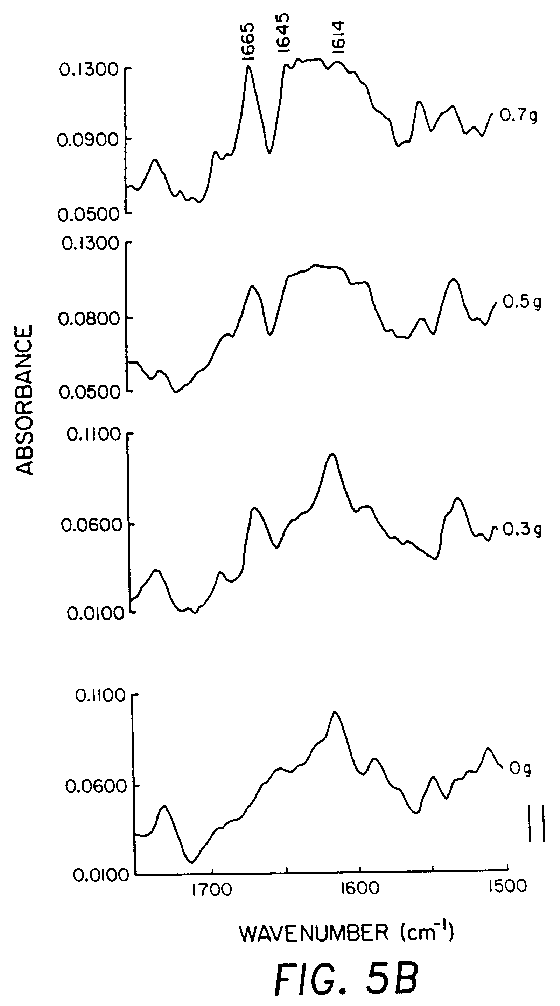

FIGURE 5 shows the deconvoluted FTIR spectra of silk fiber from the minor ampullate gland of Nephila clavipes; A): fiber axis perpendicular to the polarized incident radiation; B): fiber axis parallel to the polarized incident radiation;
FIGURE 6 shows the entire cDNA sequence and the corresponding amino acid sequence for spider Silk Protein 1 from the major ampullate gland of Nephila clavipes. The spider silk protein is encoded by the DNA through position 2154. Thus, the last amino acid is the Ser (amino acid 718) at positions 2152-2154. The cDNA sequence and the corresponding amino sequence are shown in SEQ. ID. NO. 1. The END codon TAG at positions 2155-2157 is not translated nor are the remaining codons; and
FIGURE 7 shows the entire cDNA sequence and the corresponding amino acid sequence for spider Silk Protein 2 from the major ampullate gland of Nephila clavipes. The spider silk protein is encoded by the DNA through position 1785. The cDNA sequence and the corresponding amino acid sequence are shown in SEQ. ID. NO. 3.

### DETAILED DESCRIPTION OF THE INVENTION

The genes for two different spider silk proteins, e.g., spider Silk Protein 1 and spider Silk Protein 2, have been cloned.

### SILK PROTEIN 1

The cDNA codes for a spider silk protein comprising repeating units which contain a sequence which can be represented by the following general formula
[(α)(β)]ₚ (I)
wherein α is an amorphous region which can form an α-helix when stretched, β is a β-crystalline region and p is an integer of 1 to 100, preferably 15 to 50, more preferably 18 to 22 for fragments and 25 to 100, preferably 30 to 90 for the full length protein.

The α region is preferably an alanine rich region which contains 4 to 10 A's, preferably 5 to 8 A's and more preferably 6 or 7 A's. The α region contains at least 50% alanine, preferably at least 60% alanine, more preferably about 70-80% alanine and comprises about 35 to 50%, preferably 30 to 40% of the total protein based on the total number of amino acids. The α region may contain other amino acids such as glycine and/or serine.

The β region is any sequence which forms a β-crystalline structure or which forms β-pleated sheets. The β-region preferably comprises 40-70% of the total protein, more preferably 50-60% of the total protein. The above percentages (%) refer to % of amino acids. For each repeat "p" in the above formula, the α and β regions may be the same or different.

The cDNA codes for spider silk protein which comprises repeating units which can also be represented by the following general formula
[(V)(α)(β)]ₚ (II)
wherein α, β and p are as defined above, v is a variable region and wherein the variable region (v), if present, is a region containing 0 to about 12 amino acids which usually begins with the sequence AGR. A majority of the repeating units in the spider silk protein may contain these units as defined in this paragraph. Representative sequences falling within this formula are shown in Table 1 below.

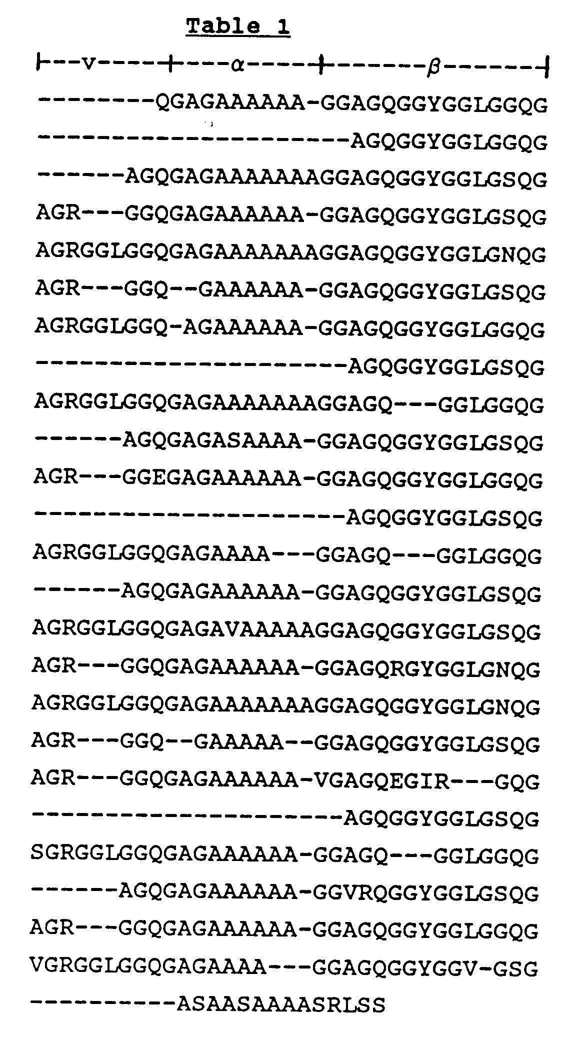

The isolated cDNA of the invention may code for a protein comprising repeating units which contain a sequence which can be represented by the following general formula
--[(A)ₘ(X)ₙ]ₚ-- (III)
wherein m is 4 to 10, preferably 5 to 8 and more preferably 6 or 7, n is 10 to 20, preferably 12 to 18 and more preferably 14 to 16, p is as defined above and each X, which may be the same or different, is selected from the group consisting of G, A, Q, Y and L, wherein at least 50% of the X's are G, more preferably at least 60% of the X's are G. For each repeat "p" in the above formula, each m and n may be the same or different.

At least 50% of the repeating units of the spider silk protein can be represented by the formula (I), (II) or (III), respectively, preferably at least 70% of the repeating units can be represented by the formula (I), (II) or (III).

The isolated cDNA of the invention contains repeating units which code for the sequence
--[(A)ₘGGAGQGGYGGLGGQG]-- (IV)
wherein m is 6 or 7.

The spider silk or fragment or variant thereof usually has a molecular weight of at least about 16,000 daltons, preferably 16,000 to 100,000 daltons, more preferably 50,000 to 80,000 daltons for fragments and greater than 100,000 but less than 300,000 daltons, preferably 120,000 to 300,000 daltons for the full length protein. The molecular weight of the spider silk protein shown in Figure 6 and listed in SEQ. ID. NO. 2 is 64,492 daltons.

In the above formulas (I)-(IV), the protein may have additional amino acids or amino acid sequences inserted into the protein in the middle thereof or at the ends thereof so long as the protein possesses the desired physical characteristics. Likewise, some of the amino acids or amino acid sequences may be deleted from the protein so long as the protein possesses the desired physical characteristics. Amino acid substitutions may also be made in the sequences so long as the protein possesses the desired physical characteristics.

The major protein from Nephila clavipes dragline silk has been cloned. The sequence comprises a repeating hexamer of Gly-Gly-X-Gly-Y-Gly, where X and Y are predominantly Gln and Ala but can be other amino acids. These repeats are separated by varying length amino acid inserts composed of Ala and Ser with small amounts of other amino acids possible. A representative sequence is as follows:
-[G-G-Q-G-A-G]-A-A-A-A-[G-G-A-G-Q-G]-G-Y-[G-G-V-G-S-G]-A-S-A-A-S-A-A-A-S-

The protein is constituted primarily by repeats of the sequence AGRGGXGGZGAG(A)₆₋₇GGAGQGGYGGLGGQG with X and Y being L, Y or Q but with X not the same as Z.

### SILK PROTEIN 2

The cDNA codes for a spider silk protein comprising repeating units which contain a sequence which can be represented by the following general formula
[(β)(α)]ₚ (I)
wherein α is an amorphous region which can form an α-helix when stretched, β is a linked β-turn region which forms a β-sheet like structure and p is an integer of 1 to 100, preferably 15 to 50, more preferably 18 to 22 for fragments and 25 to 100, preferably 30 to 90 for the full length protein.

The α region is preferably an alanine rich region which contains 4 to 10 A's, preferably 6 to 10 A's. The α region contains at least 50% alanine, preferably at least 60% alanine, more preferably about 70-100% alanine and comprises about 35 to 50%, preferably 30 to 40% of the total protein based on the total number of amino acids. The a region may contain other amino acids such as serine and/or glycine. Such substitutions may have no significant effect on function.

The β region is any sequence which forms a linked β-turn. The β-turn region is composed of repeats of GPGQQGPGGYGPGQQGPSGPGS with occasional substitutions and one insert of GGY. The β-turn region has a much higher amount of proline than the β-crystalline region of silk protein 1. It is believed that the proline is responsible for causing the turns or "kinks" in the protein. Each β-turn will usually contain 1 proline, usually from 0 to 2 prolines. Each β-region will usually contain more than 1 β-turn, usually 4 or 5 β-turns. The β-region preferably comprises 40-70% of the total protein, more preferably 50-60% of the total protein. The above percentages (%) refer to % of amino acids. For each repeat "p" in the above formula, the α and β regions may be the same or different.

The cDNA codes for spider silk protein which comprises repeating units which can also be represented by the following general formula
[(β)(α)(v)]ₚ (II)
wherein α, β and p are as defined above, v is a variable region and wherein the variable region (v), if present, is a region containing 0 to about 20 amino acids, usually 0 to about 18 amino acids, which usually contains the sequence GPGGY and/or GPGQQ. A majority of the repeating units in the spider silk protein may contain these units as defined in this paragraph. Representative sequences falling within this formula are shown in Table 2 below.

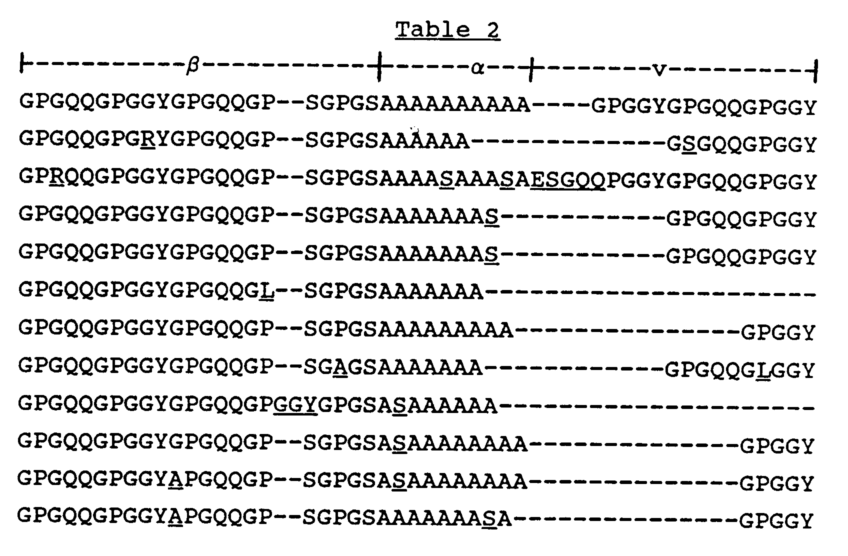

The isolated cDNA of the invention may code for a protein comprising repeating units which contain a sequence which can be represented by the following general formula
--[(A)ₘ(X)ₙ]ₚ-- (III)
wherein m is 4 to 10, preferably 6 to 10, n is 10 to 20, preferably 12 to 18 and more preferably 14 to 16, p is as defined above and each X, which may be the same or different, is selected from the group consisting of P, G, A, Q, Y and L, wherein at least 50% of the X's are G, more preferably at least 60% of the X's are G. For each repeat "p" in the above formula, each m and n may be the same or different.

At least 50% of the repeating units of the spider silk protein can be represented by the formula (I), (II) or (III), respectively, preferably at least 70% of the repeating units can be represented by the formula (I), (II) or (III).

The isolated cDNA of the invention may contain repeating units which code for the sequence
--[β(A)ₘ]-- (IV)
wherein β is as defined above and m is 6 to 10.

The spider silk or fragment or variant thereof usually has a molecular weight of at least about 16,000 daltons, preferably 16,000 to 100,000 daltons, more preferably 50,000 to 80,000 daltons for fragments and greater than 100,000 but less than 300,000 daltons, preferably 120,000 to 300,000 daltons for the full length protein. The molecular weight of the spider Silk Protein 2 shown in Figure 7 and listed in SEQ. ID. NO. 4 is 51,157 daltons.

In the above formulas (I)-(IV), the protein may have additional amino acids or amino acid sequences inserted into the protein in the middle thereof or at the ends thereof so long as the protein possesses the desired physical characteristics. Likewise, some of the amino acids or amino acid sequences may be deleted from the protein so long as the protein possesses the desired physical characteristics. Amino acid substitutions may also be made in the sequences so long as the protein possesses the desired physical characteristics.

Abbreviations for amino acids used herein are conventionally defined as described hereinbelow unless otherwise indicated.

| Amino Acid | Three-letter abbreviation | One-letter symbol |
|---|---|---|
| Alanine | Ala | A |
| Arginine | Arg | R |
| Asparagine | Asn | N |
| Aspartic acid | Asp | D |
| Asparagine or aspartic acid | Asx | B |
| Cysteine | Cys | C |
| Glutamine | Gln | Q |
| Glutamine acid | Glu | E |
| Glutamine or glutamic acid | Glx | Z |
| Glycine | Gly | G |
| Histidine | His | H |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

Recombinant spider silk protein can be recovered from cultures by lysing the cells to release spider silk protein which is present inside the cells. Initially, cell debris can be separated by centrifugation. The remaining debris and the supernatant are then repeatedly treated with solvents in which the cell debris are soluble but in which the spider silk protein is not soluble to thereby precipitate spider silk protein. These procedures can be repeated and combined with other procedures including filtration, dialysis and/or chromatography to obtain a pure product.

In accordance with degeneracy of genetic code, it is possible to substitute at least one base of the base sequence of a gene by another kind of base without causing the amino acid sequence of the polypeptide produced from the gene to be changed. Hence, the DNA of the present invention may also have any base sequence that has been changed by substitution in accordance with degeneracy of genetic code. For example, the amino acid sequence coded by a modified DNA corresponding to Figure 6 obtained by the above-mentioned substitution is identical with the amino acid sequence of Figure 6.

The DNA is readily modified by substitution, deletion or insertion of nucleotides, thereby resulting in novel DNA sequences encoding spider silk protein or its derivatives. These modified sequences are used to produce mutant spider silk protein and to directly express spider silk protein.

DNA regions are operably linked when they are functionally related to each other. For example, DNA for a presequence or secretory leader is operably linked to DNA for a polypeptide if it is expressed as a preprotein which participates in the secretion of the polypeptide; a promoter is operably linked to a coding sequence if it controls the transcription of the sequence; or a ribosome binding site is operably linked to a coding sequence if it is positioned so as to permit translation. Generally, operably linked means contiguous and, in the case of secretory leaders, contiguous and in reading phase.

Suitable host cells are prokaryotes, yeast or higher eukaryotic cells. Prokaryotes include gram negative or gram positive bacteria, for example E. coli or Bacilli. Higher eukaryotic cells include established cell lines of insect, spider or mammalian origin as described below.

Prokaryotic host-vector systems are preferred for the expression of spider silk protein. A plethora of suitable microbial vectors are available. Generally, a microbial vector will contain an origin of replication recognized by the intended host, a promoter which will function in the host and a phenotypic selection gene, for example, a gene encoding proteins conferring antibiotic resistance or supplying an auxotrophic requirement.

Vectors must contain a promoter which is recognized by the host organism. This is generally a promoter homologous to the intended host. Promoters most commonly used in recombinant DNA construction include the β-lactamase (penicillinase) and lactose promoter systems, a tryptophan (trp) promoter system and the tac promoter. While these are the most commonly used, other known microbial promoters are suitable. Details concerning their nucleotide sequences have been published, enabling a skilled worker operably to ligate them to DNA encoding spider silk protein in plasmid vectors and the DNA encoding spider silk protein. At the present time a preferred vector is pGEM3Z. Other possible expression vectors are λ GT11 and pGEM5Zft.

In addition to prokaryates, eukaryotic microbes such as yeast cultures may be transformed with spider silk protein encoding vectors. Saccharomyces cerevisiae, or common baker's yeast, is the most commonly used among lower eukaryotic host microorganisms, although a number of other strains are commonly available. Yeast vectors generally will contain an origin of replication from the 2 micron yeast plasmid or an autonomously replicating sequence (ARS), a promoter, a DNA sequence coding for spider silk protein, sequences for polyadenylation and transcription termination and a selection gene.

Suitable promoting sequences in yeast vectors include the promoters for metallothionein, 3-phosphoglycerate kinase or other glycolytic enzymes.

Other promoters, which have the additional advantage of transcription controlled by growth conditions, are the promoter regions for alcohol dehydrogenase 2, isocytochrome C, acid phosphatase, degradative enzymes associated with nitrogen metabolism, and the aforementioned metallothionein and glyceraldehyde-3-phosphate dehydrogenase, as well as enzymes responsible for maltose and galactose utilization. In constructing suitable expression plasmids, the termination sequences associated with these genes are also ligated into the expression vector 3' of the spider silk protein coding sequences to provide polyadenylation of the mRNA and termination.

In addition to microorganisms, cultures of cells derived from multicellular organisms may also be used as hosts. In principal, any higher eukaryotic cell culture is workable, whether from vertebrate or invertebrate culture. For example, an insect virus such as Baculovirus can be used to express silk protein in insect cells. However, interest has been greatest in vertebrate cells, and propagation of vertebrate cells in culture (tissue culture) has become a routine procedure in recent years. Examples of useful host cell lines are VERO and HeLa cells, Chinese hamster ovary (CHO) cell lines, and WI38, BHK, COS-7 and MDCK cell lines. Expression vectors for such cells ordinarily include (if necessary) an origin of replication, a promoter located upstream from the gene to be expressed, along with a ribosome binding site, RNA splice site (if intron-containing genomic DNA is used), a polyadenylation site, and a transcriptional termination sequence.

The transcriptional and translational control sequences in expression vectors to be used in transforming vertebrate cells are often provided by viral sources. For example, commonly used promoters are derived from polyoma, Adenovirus 2, and most preferably Simian Virus 40 (SV40). The early and late promoters are particularly useful because both are obtained easily from the virus as a fragment which also contains the SV40 viral origin of replication. Smaller or larger SV40 fragments may also be used, provided the approximately 250 bp sequence extending from the Hind III site toward the Bg1I site located in the viral origin of replication is included.

An origin of replication may be provided either by construction of the vector to include an exogenous origin, such as may be derived from SV40 or other viral (e.g., Polyoma, Adenovirus, VSV, or BPV) source, or may be provided by the host cell chromosomal replication mechanism. If the vector is integrated into the host cell chromosome, the latter is often sufficient.

The protein and DNA sequence of the major protein in dragline silk is described hereinbelow. The protein sequence comprises a repeating pattern which is divided into three segments. The first segment contains up to 9 amino acids. This sequence is AGRGGLGGQ but the segment can contain no amino acids or a combination of the first three with either of the second set of three. The second segment is composed of 9 or 10 amino acids with a sequence of GAGAAAAAA(A). This sequence is present in all repeats. The final segment is composed of 15 amino acids with a sequence of GGAGQGGYGGLGGQG. The variability in this segment is at the underlined position which can also be S,N or A. This third segment is found in all repeats. In all of these segments there are rare substitutions of other amino acids but no pattern is seen nor are they present in more than 5% of the repeats sequenced.

The DNA sequence for this protein shows a very high preference for A or T in the third position of the codon. This is such that only about 10% of the codons end in G or C compared to an expected 50% if it is random. This is likely a stability factor for the DNA to prevent recombination and deletion events from occurring.

Spider dragline silk has a number of unusual properties. These include a tensile strength greater than steel or carbon fibers (200 ksi), elasticity as great as some nylon (35%), a stiffness as low as silk (0.6 msi), and the ability to supercontract in water (up to 60% decrease in length). These properties are unmatched by any other material. The new material of the invention would provide these features in a very low weight material. The cloned protein of the invention is the major component for this silk.

Spider silk, especially dragline silk, has a tensile strength of over 200 ksi and yet has an elasticity of nearly 35%. This combination results in it having the greatest energy input necessary to break any known fiber including Kelvar and steel. This combination is also unique in both biological and man-made materials. When spun into fibers, which can be done by dissolving spider silk in an appropriate solvent and forcing it through a small orifice, spider silk can have numerous uses. For example, one large volume use is for clothing. Silk with elasticity would have a unique place in the market even at high prices. It may also be applicable for certain kinds of high strength uses such as rope, surgical sutures, flexible tie downs for certain electrical components and even as a biomaterial for implantation (e.g., artificial ligaments or aortic banding). Thus, there are numerous applications for the invention including high-tech clothing, rope, sutures, medical coverings and others where various combinations of strength and elasticity are required. It is also possible to modify the properties of the silk fibers by altering the protein sequence.

The fibers may be used for the same uses as natural spider silk fibers. The fibers may also be mixed with various plastics and/or resins to prepare a fiber-reinforced plastic and/or resin product. Because spider silk is stable up to 100°C, the fibers may be used to reinforce thermal injected plastics.

The present invention is also directed to a method for preparing variants of natural spider silk protein which comprises determining the DNA sequence of a gene which codes for natural spider silk protein, preparing a variant of said DNA sequence, and expressing said variant of said DNA sequence in a cell or microorganism to produce a variant spider silk protein having properties different form the properties of natural spider silk. The variants of natural spider silk DNA may be prepared by identifying regions of said DNA of said gene which correspond to amorphous regions in said natural spider silk protein and modifying said DNA sequence corresponding to said amorphous region or regions to change the elasticity characteristics of the protein expressed by said variant DNA or identifying regions of said DNA of said gene which correspond to β-crystalline regions in said natural spider silk protein and modifying said DNA sequence corresponding to said β-crystalline region or regions to change the strength characteristics of said protein.

Based on the amino acid sequence of peptides derived from Nephila dragline silk, DNA probes are used to identify several clones from a silk gland cDNA library. More specifically, over 2kb of two separate clones have been sequenced in the manner described in Example 3 for the Nephila dragline silk protein. The largest of these clones (2.5kb) has been fully sequenced. The sequence contains the poly A site, 340 bases of the 3' untranslated region and the remainder a protein coding region. The protein region contains a basic 34 amino acid repeat. The repeat itself contains three regions. The first comprises 0-9 amino acids with a sequence of AGR(GGX)₂. Clearly this region is not highly conserved. The second region has a sequence of GAG(A)ₓ which is highly conserved in all repeats and is 8-10 amino acids long. The third segment is (GGX)₅ and is 15 amino acids long and is very highly conserved. In most cases, X is A, Q, Y or L. Clones for other silk proteins have been isolated and sequenced. In addition, the amino acid sequence from several spider silk proteins have been determined and these include: Nephila dragline (GYGPG, GQGAG, GAGQG, GYGGLG) and cocoon (SAFQ) and Araneus dragline (GPYGPGQQGP) and cocoon (FLGG, SVGLVL/I -A-Y-A-L). Over 18 positive clones have been identified from a Nephila silk gland library using an 18 mer probe based on the dragline protein sequence.

In accordance with the invention, the recombinant protein can be made in bacteria, purified, if necessary, and spun into fibers. The optimum protein size for production may be determined so that the protein still retains the important physical properties.

The sequences of the spider silk protein repeats may vary in many ways and yet still be within the scope of the invention. For instance, Gln, Leu, and Tyr may be substituted for each other in the sequence. Moreover, the removal of poly-Ala segments results in a silk having a lower elasticity. Furthermore, additional variety in the protein sequence may result in replacing one Gly in each pair of Glys in the GGX with a Ser.

Accordingly, the silk protein of the invention can be varied depending upon its intended use. For example, when lower elasticity is desirable, the poly-Ala stretch of the protein sequence should be removed. In contrast, when a high degree of elasticity is desired, the length of the poly-Ala stretch should be increased. Also, if a less stiff silk is desired, glycine should be substituted with serine.

In accordance with the present invention, large quantities of protein having the desired properties can be obtained. This protein can be made into fibers for any intended use. Clones may also be sequenced for making minor ampullate, cocoon and swathing silks.

Mixed composites of fibers are also of interest due to their unique properties. Such mixed composites confer flexible behavior into otherwise stiff materials and would provide strength at the same time.

The following Examples are intended to illustrate the claimed invention and will enable others skilled in the art to understand the invention more completely. However, the invention should not be interpreted as being limited to only these representative Examples.

### EXAMPLE 1

The major and minor silks from the spiders Nephila clavipes are harvested as described in Work, R.W., et al. J. Arachnol., 10, 1-10 (1982), and then allowed to completely dry at room temperature. A length of 3-5 cm of silk is used for each FTIR experiment. Spectra are obtained with an Analect FX 6260 FTIR spectrometer through an Analect Micro-XA FTIR microscope at 2 cm⁻¹ resolution. Interferograms of 128 scans are routinely obtained and spectra are partially deconvoluted by the method of Kauppinen, J.K., et al., Appl. Sectrosc. 35, 271-276, (1986), employing Gaussian components with a half width at half height of 12 cm⁻¹ and a resolution enhancement factor (K) of 2. Axial extension experiments are carried out by fixing one end of the silk and attaching the other end to small suspended weights of the indicated size.

Before carrying out the actual experiments, the uniformity of the silk fiber is examined by taking FTIR spectra of several randomly selected sites along the fiber, both parallel and perpendicular to the polarized light. The spectra differed from each other by less than 2 cm⁻¹ in terms of peak positions (results not shown). Thus, the silk fiber is shown to be spectrally homogeneous.

To examine the possibility of structural changes in major ampullate silk of the spider Nephila clavipes when the protein responds to axial strain, FTIR spectra of individual silk fibers are obtained through an infrared microscope. Original and partially deconvoluted infrared spectra of silk fibers, with the IR radiation polarized perpendicular to the fiber axis, are shown in Fig. 1C and Fig. 1D, respectively, as a function of axial tension on the fiber. Strong perpendicular dichroism in the amide 1 band is found for peaks at 1694, 1630 and 1620 cm⁻¹, consistent with the high content of β-structure previously detected in other forms of silk by IR spectroscopy, Suzuki, E. , Spectrochim. Acta., 23A, 2303-2308 (1987); Fraser, R.D., et al., Conformation in Fibrous Protein, Academic Press, New York and London (1973). Weak bands at 1657 and 1675 cm⁻¹ are also evident and can be assigned to disordered regions and anti-parallel β-sheet respectively, Fraser, R.D.B., et al., Conformation in Fibrous Protein, Academic Press, New York and London (1973); Byler, D.M., Biopolymers, 25, 469-487 (1986). Amide II bands are seen at 1520 and 1550 cm⁻¹ indicating β-sheet and a very small amount of helical region respectively, Fraser, R.D.B. Conformation in Fibrous Protein, Academic Press, New York and London (1973); Cantor, C.R. et al, Biophysical Chemistry, Part II, 466-472, Freemnan, San Francisco (1980). When tension is applied to the silk fiber the radiation perpendicular to the fiber axis shows only minor changes in the Amide I region. In contrast, the peak near 1550 cm⁻¹ in the Amide II region decreases in intensity upon applied tension and shifts to 1557 cm⁻¹.

Strikingly different results are observed when the radiation is oriented parallel to the fiber axis (Fig. 1A and Fig. 1B). While all of the β-structure peaks are still clearly evident and their position unchanged, application of tension produces a dramatic increase in the absorbance at 1651 cm⁻¹. A peak in this region which displays parallel dichroism is strongly indicative of an α-helical structure, Fraser, R.D.B., et al., Conformation in Fibrous Protein, Academic Press, New York and London (1973); Byler, D.M., et al., Biopolymers, 25, 469-487 (1986). A similar increase is seen in the Amide II band at 1559 cm⁻¹ which also indicates the formation of helix. A band at 1512 cm⁻¹ is formed as well and splits into two components at 1508 and 1517 cm⁻¹ under tension. An absorbance in this region in proteins is usually assigned to either nonhydrogen-bonded peptide groups, Cantor, C.R., et al., Biophysical Chemistry, Part II, 466-472 (1980), Freemnan, San Francisco, or tyrosine residues, Fraser, R.D.B., et al. Conformation in the Fibrous Protein, Academic Press, New York and London (1973). The tyrosine content of this fiber is only 3-4%, so the latter assignment seems less likely, which also suggests a conformational change is induced by the tension. Additional confirmation that the observed changes are involved in the elastic behavior of drag-like silk comes from the complete return of the original spectra when tension is released. Two phases can be distinguished in the relaxation process (Fig. 2). During the first phase (0-1 hr after tension release) the spectra show the silk structure quickly but not completely returning toward its original form. In the second phase (1-12 hrs. after tension release) the relaxed silk gradually assumes the complete initial conformation.

### EXAMPLE 2

Example 1 is repeated on silk from the major ampullate gland of another spider species Araneus gemmoides which is extracted in the same manner as described in Example 1. The spectra of a silk fiber with fiber axis perpendicular and parallel to the polarized light are shown in Fig. 3. As seen previously there is a peak around 1650 cm⁻¹ which appears as tension is applied and the silk fiber is parallel to the polarized incident radiation. Furthermore, in the parallel spectra of the major ampullate silks the peak around 1645 cm⁻¹ from unordered structure is replaced by an α-helical signal as axial tension is applied.

To investigate the possibility that the α-helical structure formed by stretching originates from randomly oriented α-helices preexisting prior to applied tension and that the applied force merely reorients the helices into a parallel array, FTIR spectra of silk fiber are measured with unpolarized light. Several strands of silk are randomly oriented on the sample plate without applied tension and spectra are obtained. The result is shown in Fig. 4. No evidence for α-helix is found in these spectra as should be seen if preexisting α-helices are present in the relaxed state.

An additional test of this hypothesis is performed by examining the spectra of silk fibers from the minor ampullate gland. The silk produced form the minor ampullate is distinct from that of the major gland in terms of amino acid composition, function and mechanical properties, Anderson, S.O. Comp. Biochem. Physiol., 35, 705-711 (1970); Work, R.W., et al., J. Arachnol., 15, 65-80 (1987); Work, R.W., Text. Res. J., 47: 650-662 (1977); Tillinghast, E.K., et al., Ecophsiol. of Spiders, 203-210 (1987), Springer, Heideberg). In particular, this silk is significantly less elastic and has a lower tensile strength than that of major ampullate gland silk, Work, R.W., J. Text Res. 47: 650-662 (1977); Lucase, F., et al., J. Text Res., 46: T440-T452 (1955). Therefore, the two different types of silk fibers are compared in terms of conformational features and molecular responses to tension employing FTIR. The spectra of silk from the minor ampullate gland with its fiber axis perpendicular and parallel to incident polarized infrared radiation are shown in Fig. 5. The spectra of major and minor ampullate silks with the fiber axis parallel to the polarized light are very different. Of particular interest is the observation that no evidence of α-helical formation can be detected in the spectra of the less elastic silk when tension is applied. Instead, a peak at 1665 cm⁻¹ appears which suggests an increase in turns in minor ampullate silk upon the very limited stretching which occurs.

The structural basis of spider silk elasticity is also examined by obtaining partial primary structure information. The dragline silk proteins from both Nephila clavipes and Araneus gemmoides are partially digested by limited acid hydrolysis, the resulting peptides are isolated by reversed-phase HPLC and sequenced by gas phase Edman-degradation, Hewick, R.M., et al., J. Biol. Chem., 256, 7990-7997 (1981). Peptides with a sequence of GQGAG and GAGQG are found to be the most common with a peptide of sequence GYGGLG nearly as common in Nephila clavipes. Based on analogy with Bombyx mori fibroin, the peptides containing alternating glycine residues presumably form the β-sheet regions, Lucas, F., et al., Comprehensive Biochem., 26B, 475-558 (1968). Partial sequencing of Araneus gemmoides reveal the Gly-rich repetitive peptides which have high propensity to form β-sheet as well. Most intriguingly, tri- and tetra- peptides containing primarily alanine residues are also observed in both species, suggesting that they are most likely present in more irregular conformations in the amorphous regions.

The combination of FTIR and peptide sequencing suggests a molecular mechanism for the elasticity of spider silk. The simplest interpretation of the FTIR results is that tension along the fiber leads to the formation of helices (probably of the alpha type). Sequencing of spider silk peptides demonstrates the similarity to other forms of silk with alternating glycine residues in β-sheet regions. There are also other structurally less well defined regions, which in the particular case of spider silk appear to be alanine rich. Short polypeptides rich in alanine have a strong tendency to form α-helices, Yang, D.S.C., et al., Nature, 333, 232-237 (1988). It has also been shown that stretching fibers of polyalanine leads to the formation of a regular α-helical structure, Bamford, C.H., et al., Nature, 173, 27-31 (1954). Recently, short alanine-based peptides are found to have the propensity to form unusually stable α-helical structure and individual alanine residues are thought to possess high helical potential, Marqusees, et al., Proc. Natl. Acad. Sci. USA, 86, 5286-5290 (1989). Thus, the alanine rich segments in the amorphous regions of major ampullate dragline silk are very likely to be the source of the observed tension induced formation of α-helix.

Major ampullate spider silk can be most simply pictured as semicrystalline regions of interlocking β-sheets which give the fiber its remarkable strength. These regions appear to change little when force is applied along the fiber axis. The β-sheet portions of individual polypeptide chains are interspersed with short, alanine-rich domains which are disordered when the fiber is in a relaxed state. Application of tension induces these regions to become helical with the energy for helix formation arising at least partially from the applied mechanical forces. This force-induced formation of ordered structure is a unique finding and may be of general relevance to other biochemical systems. This clearly contrasts with the α to β transformation seen upon stretching in some other silks, (Lucase, F., et al., Comprehensive Biochem., 26B, 475-558 (1968). When tension is relaxed the ordered regions are then entropically driven back to a more disordered state producing the observed elasticity.

### EXAMPLE 3

### 1. Purification and identification of silk proteins

The spiders in this research are Nephila clavipes purchased from Marine Specimens Ltd., Florida. The first step to be taken is to obtain pure silk from a single type of silk gland. Using the method described by Work et al., J. Arachrol., 10, 1-10, (1982), an apparatus was designed to draw a single silk fiber from one spinnerette of the spider. The single silk fiber is stuck on a spool. A variable speed electrical drill is then used to forcibly remove 0.5-1 mg of pure silk from the spinnerette. The pure silk is dissolved in 5M LiSCN, TFA (trifluoroacetic acid) 100% at room temperature and then is run on reverse phase HPLC (high performance liquid chromatography), using a C-18 column with buffer A containing 0.4M acetic acid/pyridine, pH 4.0 and buffer B which consists of buffer A containing 40% propanol. Two peaks of peptide are observed by fluorescence.

### 2. Amino acid composition of the protein(s)

The pure silk is hydrolyzed in 6N HCl for 35 min at 155°C under vacuum to prevent oxidation. After drying and removal of HCl, the sample is analyzed by the OPA (O-phthalaldehyde) (Jones, et al., J. Liq. Chromat., 4, 565-586 (1985) and the PITC (phenyl isothiocyanate) (Heinrickson, R.L., et al. Anal. Biochem., 136, 65-74 (1984) methods with HPLC on a C-18 reverse-phase column.

### 3. Protein cleavage and fragment purification

The preliminary results show a "ragged" amino terminus of the whole silk protein. Thus, either the proteins in the silk do not have the same sequences or their N-terminal sequences do not start at the same place. Protein cleavage and fragment purification are conducted to provide the fragments of silk protein for partial sequencing. The fragments are cleaved by 6M HCl hydrolyzing under a temperature of 155°C for 3 min. The fragments are purified by running on HPLC using a C-18 reverse-phase column.

| Time speed | Volume of B-buffer % | Record |
|---|---|---|
| 0 | 0 | 10 cm/60 |

| min | | |
|---|---|---|
| 20 min | 6.6 | |
| 40 min | 13.2 | flow |

| volume | | |
|---|---|---|
| 60 min | 19.8 | 0.75 |

| ml/min | | |
|---|---|---|
| 80 min | 33.3 | |
| 100 min | 50 | |
| 120 min | 66.6 | |
| 140 min | 100 | |

### 4. Partial amino acid sequencing of protein

The pure fragments are sequenced by an Applied Biosystem gas phase protein sequencer (470A) based on an amino acid analysis of the fragments, which provides the information to determine if the fragments could represent a suitable sequence for a DNA probe such as GQGAG, GAGQG or GYGGLG.

### 5. Construction of synthetic DNA probe

Synthesis of synthetic DNA probes is conducted using an automated Applied Biosystems DNA synthesizer (430A). The product DNA can be utilized as the hybridization probes. Since glycine and alanine are the major components of the protein, using four different codons for these amino acids at the third position increases the possibility of matching the most frequent codon on these fragments.
5'CCnCGnCCnGT(C or T)CC3'
n=ACGT, four different bases.

### 6. cloning cDNA from silk gland mRNA

In order to obtain mRNA from silk glands, the spiders are forcibly silked to stimulate mRNA synthesis, Canceles, G.C., et al., J. Exp. Zoo., 216, 1-6 (1981). The major ampullate silk glands are dissected from the abdomen of the spiders (picture of anatomical location of the silk glands is from the book, Foelix, F.R., Biology of Spider) and immersed in liquid nitrogen immediately. After adding a small amount of liquid nitrogen and silk glands into a pestle and mortar, the glands are ground to powder. RNA is extracted by the SDS hot phenol method, Taylor, D.W., et al., Mol. Biochem. Parasitol, 10, 305-318 (1984). An oligo dT column is used to isolate the mRNA from the total RNA, Haim Aviv et al., PNAS, 69; 1408-1412 (1972).

The reverse transcription of the mRNA to cDNA is done using the RiboClone cDNA Synthesis System from Promega (Technical Manual). After making cDNA, the radioactively labeled cDNA is run through a Sepharose 4B gel filtration column to separate large fragments from small fragments. cDNA's larger than 500 bp are ligated into the vectors and transformed into XL1-blue E. coli to construct a cDNA library (Maniatis, T., et al., Molecular Cloning, A Lab Manual (1982)).

The pBluescriptSK plasmids which have the function of producing single strand DNA under the trigger of helper phages (Manual from Stratagene) are used as the vectors to clone the cDNA. The pBluescriptSK plasmids from Stratagene have been large scale produced (Large prep using the Triton lysis method, Frederick M. Ausubel, et al., Current Protocols in Molecular Biology, Volume 1, 1987), lysozyme incubation time was 20 min at 37°C and purified by CsCl gradient in an ultracentrifuge (L7-55, Beckman)(Frederick M. Ausubel, et al., Current Protocols in Molecular Biology, Volume 1, 1987, published by Greene Publishing Associates and Wiley-Interscience) using vti80 rotor at 24°C, 54,000 rpm overnight. Further plasmids clean-up is done by SDS sucrose gradient, Maniatis, T., et al., Molecular Cloning, A Lab Manual (1982), published by Cold Spring Harbor.

The pBluescriptSK plasmids are cut by restriction enzyme Sma1 to create a blunt end. In order to reduce the self-ligation rate of the plasmids, alkaline phosphatase from calf intestine (CIP) (Mannheim Boehringer) is used to remove the phosphate at the 5-end of the plasmids (Maniatis, T., et al., Molecular Cloning, A Lab Manual (1982) published by Cold Spring Harbor). Deactivation of CIP was conducted at 75°C instead of 68°C for 30 min. and Elutip-d column (Schleicher and Schuell, Manufacturer, Manual for purification of DNA) was used to purify and clear the vectors.

Ligation of cDNA with pBluescriptSK is carried out at 4°C overnight by T4 DNA ligase (Maniatis, T., et al., Molecular Cloning, A Lab Manual (1982), published by Cold Spring Harbor) then transformed into competent XL-1 blue E. coli. The bacteria are inoculated overnight in 1X YT and grown up to OD₆₀₀0.3-0.6. The bacteria are spun down in a JA 20 rotor at 5000 rpm for 5 min, then resuspended in 1/2 the original volume of 50 mM CaCl₂+10mM Tris, pH 8. The solution is iced for 20 min. and the cells are spun down the same as before and resuspended in 1/20-1/50 of the original volume of 50 mM CaCl₂. Aliquot in 0.3 ml. Add DNA to the appropriate tubes. Ice for 60 min. and heat shock for 3 min. at 45°C. Spread the plates and incubate for overnight at 37°C. The colonies of bacterial cells with the plasmid which has ampicillin resistance markers survive in the YT agar plates with ampicillin.

### 7. Synthetic oligodeoxynucleotide to screen the cDNA library

The synthetic oligodeoxynucleotide probes are labeled with P³² by T4 polynucleotide kinase (Maniatis, T., et al., Molecular Cloning, A Lab Manual (1982), published by Cold Spring Harbor). The white colonies from the plates were transferred to 96 well assay plates with YT medium with ampicillin and grown again at 37°C overnight. Then the bacteria with plasmid are transferred to Hybond-N hybridization transfer membranes (Amersham) using a 32 pin stamp and allowed to grow overnight in ampicillin plates. The 32 pin stamp is a homemade stamp consisting of pins inserted into a styrofoam block to match to wells of the 96 well plate. Then plasmid numbers are amplified on chloramphenicol plates. NaOH was used to lyse the bacteria and the DNA on the membranes was fixed by baking in an oven with a vacuum at 80°C for 2 Hr (Maniatis, T., et al., Molecular Cloning, a lab manual (1982), published by Cold Spring Harbor).

The libraries were screened by a radioactively labeled oligodeoxynucleotide probe using the method of Wood and Lawn because of the highly complex DNA structure, Wood, W.I., et al., PNAS, 82, 1585-1588 (1985).

### 8. Applying Southern transfer and hybridization of the DNA to confirm positive colonies from the first screening.

Positive colonies are picked and the grown in YT medium and the plasmid DNA with inserts are extracted by mini preparation. (Promega Catalog and Frederick M. Ausubel, et al., Current Protocols in Molecular Biology, Volume 1, 1987). After running the DNA in the agarose gel, the samples are blotted onto N-membranes (Maniatis, T., et al., Molecular Cloning, A Lab Manual (1982), published by Cold Spring Harbor) and hybridized again by the same probes as above, Wood, W.I., et al., PNAS, 82, 1585-1588, (1985).

### 9. Restriction enzyme digestion DNA of the positive colonies.

Since there was more than one colony showing positive by the probe hybridization, restriction enzymes including BamHl, Ecor1, Pst1, Hae3, Apal, Clal, Hinc2, Hind3, Kpn1, Sal1, Sal2, Xho1, Ssp1 and Sph1 are applied (Digestion conditions for each enzyme according to the manufacturer instruction) to detect the differences between these positive colonies as well as to obtain information for further DNA sequencing analysis.

### 10. Subcloning the restriction enzyme digested fragments and screening by the probe.

In order to avoid wasting time on sequencing of suspicious colonies, the fragments digested by Hae III and Pst1 were filled by four different dNTP ACGT using a Klenow fragment of E. coli DNA polymerase (Maniatis, T., et al., Molecular Cloning, A Lab Manual (1982)) to create blunt-end fragments. Then the fragments were randomly subcloned into an M13 phage by the same procedure as cloning. The plaques were screened again by the probes (as above). The positive plaques were probed again by southern hybridization, Wood, W.I., et al., PNAS, 82, 1585-1588, (1985).

### 11. Sequencing the positive subclone fragments.

Positive plaques of M13 phages with inserts were cultured at 37°C with BSJ 72 E. coli overnight to produce single strand DNA for DNA sequencing. For purification of single strand DNA, 1 ml phage containing the supernatant mix with 0.25 ml RNAase/PEG(PEG 10%, NaCl 2.5M, EDTA 0.015M) standing for 2-3 hr. at room temperature. Spin 5 min. in microfuge to pellet phage. Remove solution as much as possible. Dissolve the pellet in 0.07 ml proteinase solution (0.01M Tris pH8, 0.001M EDTA pH8, 0.2% Sarkosyl, 0.07 mg/ml Proteinase K), incubation 20 min. at 55°C, add 0.05 ml 0.25M NaCl. Extract with 0.15 ml water saturated Phenol, extract with 0.130 ml Phenol/CHCl/Isoamyl Alcohol (50/45/5%), then extract with 0.120 ml CHCl. Precipitate with 0.2 ml EtOH at -80°C more than 20 min. or -20°C more than 1 hr. Spin 15 min. then wash the pellet with 70% EtOH, dry pellet and resuspend in 40 ml H₂O. After purification of single strand DNA, the universal primer for an M13 phage was used to hybridize the template and the DNA was sequenced based on the method of, Sanger, F., et al., PNAS, 74: 5463-5467 (1977). S³⁵-dATP was used as labelling for an exposure picture of the sequencing. The apparatus of sequencing Gel Electrophoresis System (Model S2) (obtained from BRL, Bethesda Research Laboratories Life Technologies, Inc.) is operated in accordance with the manufacturer's instructions. Running condition: 70 Watts Time: 3-7 hours.

The larger gel plate is siliconized. The glass is then baked 4 hours at 90°C, only when the glass is new or washed by NaOH. Each time the larger plate was siliconized without baking before using.

The smaller plate is treated with 2 ml of a 2% solution in ethanol of 3-Methacryloxypropyl-trimethoxysilan(Sigma) each time before using. Both larger and smaller plates were thoroughly cleaned with detergent and distilled water. 90% ethanol was used to do final cleaning before being siliconized, treatment of 3-methacryloxypropyl-trimethoxysilan and filling gel.

7% Acrylamide/bis, 8M Urea gel was used all the time.

| For 500 ml stock Acrylamide solution | |
|---|---|
| Acrylamide | 33.2 g |
| Bis-acrylamide | 1.75 g |
| Urea | 240 g |
| Solution should be deionized by Amberlite MB-3 Monobed Resin(Sigma), 25g for 500 ml solution. | |

| For 1 liter 10x sequencing buffer TBE | |
|---|---|
| Tris base | 160 g |
| Boric Acid | 38 g |
| Disodium EDTA | 9 g |
| 1x TBE buffer is the working condition mixing with 7% acrylamide/bis/urea solution. 15% Ammonium persulphate (0.20 ml) and TEMED (0.05 ml) were used to catalyze 60 ml. total volume of gel for polymerization. | |

### 12. Sequencing the colonies which have a sequence which hybridizes with the probes.

Three colonies are sequenced using the method of Sanger, F., et al., PNAS, 74, 5463-5467 (1977). S³⁵-dATP was used as labelling for exposure picture of the sequencing. Because the sizes of these colonies are different, ranging from 800 bp to 2.4 k BP, each reaction of sequencing only provided clear reading of 300 to 350 BP. In order to read the whole sequence of the DNA, a kit for the partial deletion DNA from Promega, the "Erase-a-Base System", was used to create different sizes of DNA for sequencing (Technical Manual, "Erase-a-Base System" Promega).

### 13. Sequencing 2.4 kb DNA of spider silk protein.

The partial sequencing results from the 2.4 kb DNA showed a repetitive sequence and high GC complex structure which can cause the fragments to delete and religate. In order to obtain correct sequence information, restriction enzyme Hae III was used to cut the 2.4 kb into small fragments varying from 150 to 900 bp and these Hae III fragments were separated by 1% low melting point agarose (Bethesda Research Laboratories) then purified by hot phenol, Maniatis, T., et al., Molecular Cloning, A Lab Manual (1982), and Elutip-d. The pure fragments in different sizes were subcloned (same method as cloning) into pBluescript KS(+/-) plasmids and M13 phages mp 18 as well as mp 19 (from Strategene) respectively for sequencing.

### 14. Northern blotting and hybridization to determine the size of mRNA for silk proteins.

mRNA is purified by running whole RNA through the oligo-dT affinity column (same as above) and through a denaturing formaldehyde agarose gel, Frederick M. Ausubel, et al., Current Protocols in Molecular Biology, Volume 1, 4.9.5-4.9.8 (1987). The mRNA were blotted onto Zeta-probe membranes, Maniatis, T., et al., Molecular Cloning, A Lab Manual (1982). Hae III digested fragments were separated by agarose gel electrophoresis (same procedure as above). Nick translation kit (Nick Translation reagent Kit, Bethesda Research Laboratories) was used to make radioactive labeled probes using the 900 bp fragment as a template. Membrane with mRNA was hybridized at 75°C by the probes to determine the size of mRNA of the silk proteins, Bio-Rad, Instruction Manual 4.3, Zeta-probe Blotting Membranes.

The DNA sequence and the corresponding amino acid sequence of the silk proteins are shown in Figure 6.

### EXAMPLE 4

A clone of spider silk DNA (2.0kb) (See Figure 6) in pBluescriptSK+/-plasmids is selected. An insert is placed in the SmaI site. The pBluescriptSK+ vector is digested by SmaI restriction enzymes in the same manner as #6 in Example 3.

The insert is cut out from pBluescriptSK+ at the BamHI and EcoRI sites, ("NEB", New England Biolabs). The EcoRI site is filled in with a Klenow Fragment (Promega, Inc. supplied the fragment and protocol) to provide a blunt end at the EcoRI site. The BamHI overhang is left to insure proper orientation of the insert into the new vector pGEM3Z. This pGEM3Z vector is prepared by digesting with BamHI (BamHI digestion is carried out at 37°C for 30 min. using the BRL reaction buffer (Bethesda Research Labs)) leaving an overhang and HincII (NEB) blunt end. The two plasmids are ligated using T-4 DNA ligase. T-4 DNA ligation is carried out at 4°C overnight using 10x ligation buffer (Maniatis, T., et al., Molecular Cloning, A Lab Manual (1982)).

GATATCGAATTCCTGCAGCCCCAGG.....................GGGATCCACTAG
TTCTAGA
EcoRI
BamHI
pBluescript
EcoRi/BamHI cut
GATATCGAATTCCT.....................GGATCC..........
CTATAGCTTAAGGA.....................CCTAGG..........
AGGTCGACTCTAGAGGATCCCCGG
TCCAGCTGAGATCTCCGAGGGGCC
pGEM3Z

Transformation of the ligated DNA is performed by adding the DNA to previously made competent cells. After incubation of cells and DNA for 2 hours at 0°C, the cells and DNA are combined with 0.85% saline soft agar (3 ml of soft agar) and poured onto LB agar plates supplemented with 40 µg/ml X-gal, 50 µg/ml ampicillin and 40 µg/ml IPTG (isopropyl β-D-trisgalactopyranoside). X-gal (5-bromo-4-chloro-3-indolyl-β-D-galactopyranoside), ampicillin and IPTG are growth additives which are determined to be necessary for adequate growth of the transformants (Stratagene, BRL). The three host cell lines used are JM109 (Stratagene), DH5αF (Bethesda Research Laboratories), and SURE (Stratagene).

Expression of spider silk protein is induced by the addition of IPTG (Bethesda Research Laboratories (BRL)) in the growth media while culturing at 37°C. The extracted protein (50 µl) is examined by PAGE/SDS phastgel (Pharmacia). Since only a small amount of protein is produced, a reading frame shift in the plasmid is induced.

The reading frame shift is accomplished by digesting the plasmid with 1 Unit SphI (SphI digestion is carried out at 37°C for 30 min. using the Promega SphI 10x buffer (Promega, Inc.)), then cutting off the 3' overhang of 4 bases with 1 Unit T-4DNA polymerase (Promega). 3' overhang is removed by adding 1 Unit T-4 DNA polymerase at room temperature for 10 mins. The bases removed are CGTA. Self-ligation occurs in a volume of 50 µl at 4°C overnight. This plasmid is then self ligated with 1 Unit T-4 DNA ligase (New England Biolabs) to form plasmid pLM-4; and transformed as described above for the previous plasmids (Stratagene, BRL) into SURE E. coli host cells (Stratagene). This E. coli was deposited at the American Type Culture Collection, Rockville, Md., U.S.A. on March 27, 1991 under the conditions of the Budapest Treaty and was assigned Deposit No. 68567.

Expression of 20-30 mg/l spider silk protein is induced by the addition of 40 µg/ml isopropylthiogalactoside (IPTG) (BRL) in the growth media. The protein is detected by PAGE/SDS (Pharmacia). This protein has the sequence as shown in Figure 6.

The protein could be purified by centrifugation of the bacteria at 600 x g for 15 min. and disruption of the bacteria by suspension in 1M acetic acid followed by centrifugation at 3000 x g for 20 min. The pellet could be dissolved in 5% SDS(1:10 vol:vol) and the centrifugation repeated. The pellet is then treated with RNase and DNAase(0.01 mg/g) and the centrifugation repeated. The pellet is dissolved in 5M Li perchlorate and dialyzed against water(1:100 vol:vol) with 4 changes of water. The dialyzed suspension is centrifuged at 15,000 x g for 30 min. The dissolving in Li perchlorate, dialysis and centrifugation is repeated 3 times.

### EXAMPLE 5

10-20 mg of the protein silk from any of Examples 1, 2 or 4 (Predictive) are collected. The silk is dissolved to saturation in 5M Li SCN or Li perchlorate. The solution containing the dissolved silk is forced through a small gauge long needle (size 24 or smaller) and then into a 1M acetic acid solution. The fiber begins to form as it emerges from the needle.

### EXAMPLE 6

Spider Silk Protein 2 was cloned as follows. From an existing Nephila clavipes major ampullate gland cDNA library, replicate nitrocellulose filters were produced. The colonies on the filters were fixed (Maniatis et al, Molecular Cloning, Cold Spring Harbor Laboratory (1982)) and hybridized with a kinased (Maniatis et al, Molecular Cloning, A Lab Manual, Cold Spring Harbor Laboratory (1982)) degenerate probe of 14 nucleotides named Ming 1 whose sequence, based on the pentapeptide G-Y-G-P-G, is
CCNGGNCCATANCC.

Hybridization followed the procedures of Wood et al (PNAS USA, 82, 1585 (1985)) utilizing tetramethylammonium chloride. The filters were autoradiographed and the twelve darkest colonies were used to generate alkaline quick preparations that were digested with EcoRI and Bam HI. The gel was photographed after ethidium bromide staining and vacublotted to Zetaprobe (Biorad). Kinased Ming 1 was used as a probe to hybridize to the Southern blot. The clone containing the largest apparent insert, clone number six with approximately two kilobases, was used for all subsequent studies. An expression vector is constructed in the same manner as in Example 4 to form plasmid pMB-2.

The original Spider Silk 2 clone (p6B) was cut with BamH1 and Sac1 restriction endonucleases and subjected to Exonuclease III digestion for 30 sec. at 35°C. This DNA was treated with S1 nuclease and Klenow to produce a blunt ended DNA which was self-ligated (T4 DNA ligase) to form a pBluescriptSK+ plasmid with a Spider Silk 2 insert 173 bp shorter than the original clone (p6B). This plasmid, pMB2, has a DNA sequence which starts at the Sac1 site of pBluescript and continues with nucleotide number 172 (the CCC) of sequence ID. NO. 3.

The E. coli SURE cells containing the plasmid (pMB-2) were deposited at the American Type Culture Collection, Rockville, Maryland, USA on March 27, 1991 and was assigned Deposit No. 68568. Expression of a fusion protein (lac gene + spider silk protein) could be induced by addition of IPTG in the same manner as in Example 4. The protein could be purified in the same manner as in Example 4 and formed into a fiber in the same manner as in Example 5.

### EXAMPLE 7 (PREDICTIVE EXAMPLE)

To facilitate high rate expression of spider major ampullate silk in bacteria, three expression vectors are used. All these are available from New England BioLabs with the precise instruction manual. The vectors have beta-lactamase gene for screening with ampicillin, and polylinker site for cloning constructed with a part of maltose binding protein (mal E) upstream of poly-liker site as well as an alpha subunit donor of beta-galactosidase downstream of poly-liker site. Transcription is conducted by tac promoter which is regulated by IPTG. The repressor gene of latose operon is also attached in all vectors, therefore high copy number of plasmid DNA may not dilute the repressors in cytosol of E. coli cells by providing competitive tac promoter region proportional to the number of plasmid DNA in a cell. The plasmid pLM4 which codes spider Silk Protein 1 is digested with EcoRI followed by purification on an agarose gel. The vector pMAL-cRI (NEB) is digested with EcoRI as well followed by CIP (calf intestinal alkaline phosphatase, Boehringer-Mannheim) treatment, then purified on an agarose gel. Two separated fragments are ligated with T4-ligase (Promega) in the presence of 5% PEG8000 (polyethyleneglycol 8000, Sigma) for one hour at 37°C. Ligate is transformed to SURE competent cells (Stratagene) according to the instruction manual, then pored onto LB plate containing ampicillin, X-gal and IPTG. Proper oriented clones are screened by either restriction enzyme digestion or DNA sequencing. The plasmid pMH2 which codes Silk Protein 2 is digested with both BamHI and XbaI to excise out the cDNA followed by filling 5'-end overhangs with Klenow fragment of E. coli polymerase I (USB), then purified on an agarose gel as above. Expression vector, pMAL-c or pMAL-p, is digested with StuI to produce the blunt ends followed by CIP treatment and purification on an agarose gel as well. Two purified fragments are ligated with T4-ligase, and transformed in SURE competent cells. Either restriction enzyme digestion or DNA sequencing is carried out to determine the orientation of the insert. E. coli cells containing either pMAL-cRI or pMAL-c vector produce spider silk in cytosol, while E. coli cells containing pMAL-p vector excrete spider silk in peri-plasma. The former product is purified in the same manner as in Example 4, but the latter product is recovered by treating cells simply with lysozyme at the concentration of .1 mg/ml on ice for 30 min followed by centrifugation to recover supernatant. Recovered protein is a fused protein with a part of maltose binding protein. Using a part of maltose binding protein, with an affinity column which is provided from NEB with the vectors, fused protein is purified. The excess maltose binding protein is removed by digesting the fused protein with the factor Xa which recognizes and cut between the maltose binding protein and spider silk. Factor Xa is provided from NEB as well.

### EXAMPLE 8- Expression of spider silk in eukaryotic cells

### (PREDICTIVE EXAMPLE)

The baculovirus system utilizes many of the protein modifications, processing and transport systems present in higher eukaryotic cells. The majority of recombinant product is expected to be functionaly and immunologically similar to the authentic proteins. The whole baculovirus system is purchased from Invitrogen Corporation. The pAc360 fusion vector is digested with BamHI followed by filling 5'-end overhangs with Klenow fragment of E. coli polymerase I. The plasmid pLM4 which codes the spider Silk Protein 1 is digested with both EcoRV and SmaI to excise out the cDNA. After purifying fragments on an agarose gel, both fragments are ligated with T4-ligase in the presence of 5% PEG8000 at 37°C for an hour. The ligate is transformed in SURE competent cells and recovered by the standard method. After purification of the proper recombinant DNA, transfection is conducted to a cell line of the fall armyworm Spodoptera frugiperda Sf9 according to the instruction manual. A homologous recombination occurs in vivo resulting in substitution of the hybrid DNA and expression of the spider silk cDNA. Since the recombinant baculovirus contains an active polyhedrin gene promoter linked to a foreign spider silk cDNA, viral occlusions do not form. Recombinant plaques are identified by visual screening finding absence of occlusions in the formed plaques under a dissecting microscope. Detail of protocol is found in the instruction manual. Putative recombinant plaques are recovered, confirmed by hybridization with a ³²P-labeled probe, and used to produce recombinant protein.

All publications, including U.S. Patents, referred to in this application are herein incorporated by reference.

The invention being thus described, it will be obvious that the same may be varied in many ways. Such variations are not to be regarded as a departure from the spirit and scope of the present invention, and all such modifications as would be obvious to one skilled in the art are intended to be included within the scope of the following claims.

## Claims

1. A DNA molecule which codes for a Spider silk protein comprising repeating units, wherein said repeating units are comprised of a sequence represented by the formula
[(A)ₘ(X)ₙ]ₚ
wherein m is 4 to 10, n is 10 to 20, p is an integer of 1 to 100 and each X, which may be the same or different, is selected from the group consisting of G, A, Q, Y and L, wherein at least 50% of the X's are G.

2. The DNA of claim 1, which further comprises variable regions.

3. The DNA of claim 1 or 2, which codes for a protein of the formula [(α)(β)]ₚ wherein α is an amorphous region which can form an α-helix when stretched and β is a region which can form β-sheets when in a folded conformation and p is an integer of 25 to 100.

4. The DNA of claim 1 or 2, which codes for a silk protein having a molecular weight of less than 300,000 daltons, or a fragment or variant thereof which comprises repeating α and β regions, wherein α is an amorphous region which forms an α-helix when stretched and β is a region which forms β-sheets.

5. The DNA of claim 1 or 2, which codes for a silk protein comprising the amino acid sequence as shown in Figure 6 or a functional fragment thereof.

6. The DNA of claim 1 or 2, which codes for a silk protein comprising the amino acid sequence as shown in Figure 7 or a functional fragment thereof.

7. The DNA of claim 1 or claim 2, wherein p is 15 to 50.

8. The DNA of claim 1 or claim 2, wherein p is 25 to 100.

9. The DNA of claim 3, wherein the α region is an alanine-rich region which contains 4 to 10 A's.

10. The DNA of claim 3, wherein the α region contains at least 50% alanine.

11. The DNA of claim 1 which codes for a protein comprising a polypeptide having an amino acid sequence which comprises repeating units of the hexamer GGXGZG, wherein X and Z are any amino acid but are most often glutamine or alanine and repeating units having the amino acid sequence (A)ₘGGAGQGGYGGLGGQG, wherein m is 6 or 7.

12. A DNA molecule of claim 11, which codes for a protein comprising repeat units having the amino acid sequence Ala-Gly-Arg-Gly-Gly-Xaa-Gly-Gly-Zaa-Gly-Ala-Gly-(Ala)ₘ-Gly-Gly-Ala-Gly-Gln-Gly-Gly-Baa-Gly-Gly-Leu-Gly-Gly-Gln-Gly, wherein m is 6 or 7 and wherein Xaa, Zaa and Baa are leucine, tyrosine or glutamine and Xaa is not the same amino acid as Zaa.

13. A DNA molecule according to claim 12, wherein Xaa is glutamine.

14. A DNA molecule of claim 1 which codes for a protein consisting essentially of repeating units selected from the group consisting of
QGAGAAAAAAGGAGQGGYGGLGGQG,
AGQGGYGGLGGQG,
AGQGAGAAAAAAAGGAGQGGYGGLGSQG,
AGRGGQGAGAAAAAAGGAGQGGYGGLGSQG,
AGRGGLGGQGAGAAAAAAAGGAGQGGYGGLGNQG,
AGRGGQGAAAAAAGGAGQGGYGGLGSQG,
AGRGGLGGQAGAAAAAAGGAGQGGYGGLGGQG,
AGQGGYGGLGSQG,
AGRGGLGGQGAGAAAAAAAGGAGQGGLGGQG,
AGQGAGASAAAAGGAGQGGYGGLGSQG,
AGRGGEGAGAAAAAAGGAGQGGYGGLGGQG,
AGQGGYGGLGSQG,
AGRGGLGGQGAGAAAAGGAGQGGLGGQG,
AGQGAGAAAAAAGGAGQGGYGGLGSQG,
AGRGGLGGQGAGAVAAAAAGGAGQGGYGGLGSQG,
AGRGGQGAGAAAAAAGGAGQRGYGGLGNQG,
AGRGGLGGQGAGAAAAAAAGGAGQGGYGGLGNQG,
AGRGGQGAAAAAGGAGQGGYGGLGSQG,
AGRGGQGAGAAAAAAVGAGQEGIRGQG,
AGQGGYGGLGSQG,
SGRGGLGGQGAGAAAAAAGGAGQGGLGGQG,
AGQGAGAAAAAAGGVRQGGYGGLGSQG,
AGRGGQGAGAAAAAAGGAGQGGYGGLGGQG,
VGRGGLGGQGAGAAAAGGAGQGGYGGVGSG,
ASAASAAASRLSS and mixtures thereof.

15. A DNA molecule according to any claim 1 to 14, wherein the repeat units are separated by varying numbers of alanine or serine residues.

16. A DNA which codes for a Spider Silk protein comprising repeating units, wherein said repeating units are comprised of a sequence represented by the formula
[(A)ₘ(X)ₙ]ₚ
wherein m is 4 to 10, n is 10 to 20, p is an integer of 1 to 100 and each X, which may be the same or different is selected from the group consisting of P, G, A, Q, Y and L, wherein at least 50% of the X's are G.

17. The DNA of claim 16, wherein p is 15 to 50.

18. The DNA of claim 16, wherein p is 25 to 100.

19. The DNA of claim 16, which codes for a protein consisting essentially of repeating units selected from the group consisting of
GPGQQGPGGYGPGQQGPSGPGSAAAAAAAAAAGPGGYGPGQQGPGGY,
GPGQQGPGRYGPGQQGPSGPGSAAAAAAGSGQQGPGGY,
GPRQQGPGGYGQGQQGPSGPGSAAAASAAASAESGQQGPGGYGPGQQGPGGY,
GPGQQGPGGYGPGQQGPSGPGSAAAAAAAASGPGQQGPGGY,
GPGQQGPGGYGPGQQGPSGPGSAAAAAAASGPGQQGPGGY,
GPGQQGPGGYGPGQQGLSGPGSAAAAAAA,
GPGQQGPGGYGPGQQGPSGPGSAAAAAAAAAGPGGY,
GPGQQGPGGYGPGQQGPSGAGSAAAAAAAGPGQQGLGGY,
GPGQQGPGGYGPGQQGPGGYGPGSASAAAAAA,
GPGQQGPGGYGPGQQGPSGPGSASAAAAAAAAGPGGY,
GPGQQGPGGYAPGQQGPSGPGSASAAAAAAAAGPGGY,
GPGQQGPGGYAPGQQGPSGPGSAAAAAAASAGPGGY and mixtures thereof.

20. A DNA according to any preceding claim 1 to 21, wherein said silk protein is one having a molecular weight from 100,000 to 300,000 daltons.

21. A plasmid PLM-4 which is contained in ATCC Deposit No. 68567.

22. A plasmid pMB-2 which is contained in ATCC Deposit No. 68568.

23. A replicable vector containing a DNA of any claim 1 to 22, which is capable of expressing a spider silk protein or a fragment or variant thereof when introduced into a suitable host.

24. A living cell or microorganism containing the vector of any claim 20 to 23.

25. The living cell or microorganism of claim 24, which is a bacterium, preferably *Escherichia coli*.

26. A transformed cell or microorganism containing the DNA of any claim 1 to 23, which codes for spider silk protein or a functional fragment thereof, which cell or microorganism is capable of expressing spider silk protein.

27. A process for preparing recombinant spider silk proteins or functional fragments thereof, comprising the use of a DNA or vector as claimed in any claim 1 to 23 and/or a living cell or microorganism as claimed in any claim 24 to 26.

28. A process for preparing a fibre of spider silk proteins or functional fragments thereof, comprising a process as claimed in claim 27 and/or the use of proteins prepared by a process as claimed in claim 27.

29. A method for producing recombinant spider silk protein which comprises cultivating the transformed cell or micro-organism of any claim 24 to 26.

30. functional Fragment of spider silk protein, which comprises repeating α and β regions, wherein α is an amorphous region which forms an α helix when stretched and β is a region which forms β-sheets and is substantially free of natural spider silk proteins, which fragment or variant is obtainable by a process as claimed in claim 27.

31. A purified spider silk protein which is essentially a single protein which comprises repeating units which contain a sequence represented by the formula
[(A)ₘ(X)ₙ]ₚ
wherein m is 4 to 10, n is 10 to 20, p is an integer of 1 to 100 and each X, which may be the same or different, is selected from the group consisting of G, A, Q, Y and L, wherein at least 50% of the X's are G.

32. Protein as claimed in claim 31, wherein for each repeat "p" in formula [(A)ₘ(X)ₙ]ₚ, each m and n are the same, preferably 10.

33. A spider silk protein comprising repeating units, as defined in Claim 1, which contain a sequence which can be represented by the following general formula
[(α)(β)]ₚ
wherein α is an amorphous region which can form an α-helix when stretched, β is a β-crystalline region and p is an integer of 1 to 100 which is substantially free of a spider silk protein comprising the amino acid sequence shown in Figure 7.

34. A spider silk protein comprising repeating units, as defined in Claim 16, which contain a sequence which can be represented by the following general formula
[(β)(α)]ₚ
wherein α is an amorphous region which can form an α-helix when stretched, β is a region which forms a β-sheet-like structure and p is an integer of 1 to 100 which is substantially free of a spider silk protein comprising the amino acid sequence set forth in Figure 6.

35. A method for making a silk protein mixture, which comprises mixing the silk protein of claim 33 with the silk protein of claim 34.

36. A spider silk protein comprising repeating units, as defined in Claim 1, which contain a sequence which can be represented by the following general formula
[(α)(β)]ₚ
wherein α is an amorphous region which can form an α-helix when stretched, β is β-crystalline region and p is an integer of 1 to 100, wherein for each repeat "p" in the above formula, the α and β regions are the same.

37. A spider silk protein comprising repeating units, as defined in Claim 16, which contain a sequence which can be represented by the following general formula
[(β)(α)]ₚ
wherein α is an amorphous region which can form an α-helix when stretched, β is a linked β-turn region which forms a β-sheet like structure and p is an integer of 1 to 100, wherein for each repeat "p" in the above formula, the α and β regions are the same.

38. The spider silk protein of any claim 30 to 37, which has repeating α and β units wherein an α unit is an amorphous region which can form an α-helix when stretched and a β unit is a region which can form β-sheets, and wherein said protein has a molecular weight ranging from 16,000 to 300,000 daltons.

39. The spider silk protein of any claim 30 to 33, which comprises a polypeptide having an amino acid sequence comprising repeating units of the hexamer GGXGZG, wherein X and Z are any amino acid but are most often glutamine or alanine.

40. The silk protein of any claim 30 to 33, wherein X and Z are glutamine or alanine.

41. The silk protein of any claim 30 to 33, wherein the repeat units are separated by varying numbers of alanine or serine residues.

42. The silk protein of claim 39, which further comprises repeating units having the sequence (A)ₘGGAGQGGYGGLGGQG, wherein m is 6 or 7.

43. The spider silk protein of any claim 30 to 33, comprising a polypeptide having the amino acid sequence of (A)ₘGGAGQGGYGGLGGQG, wherein m is 6 or 7, or tandem repeats of this amino acid sequence.

44. The spider silk protein of claim 33, comprising a polypeptide having the amino acid sequence Ala-Gly-Arg-Gly-Gly-Xaa-Gly-Gly-Zaa-Gly-Ala-Gly-(Ala)ₘ-Gly-Gly-Ala-Gly-Glu-Gly-Gly-Baa-Gly-Gly-Leu-Gly-Gly-Glu-Gly, wherein m is 6 or 7 and wherein Xaa, Zaa and Baa are leucine, tyrosine or glutamine and Xaa is not the same amino acid as Zaa.

45. The spider silk protein of claim 33, comprising a polypeptide having the amino acid sequence GAAAAAA(A) linked by a peptide bond to the amino terminus of a polypeptide having the amino acid sequence GGAGQGGYGGLGGQG, wherein Y is tyrosine.

46. A silk protein of any claim 30, 34 and 37, which comprises a polypeptide having an amino acid sequence comprising repeating units having the amino acid sequence GPGQQGPGYYGGQQGPSGPGS.

47. The spider silk protein of any claim 30, 34, 37, and 46 comprising a polypeptide having the amino acid sequence GPGQQGPGGYGPGQQGPSGPGS linked by a peptide bond to the amino terminus of a polypeptide having the amino acid sequence GAGAAAAAA(A), or tandem repeats of the amino acid sequence of said linked polypeptides.

48. The spider silk protein of any claim 30, 34, 37, and 46 comprising a first polypeptide having the amino acid sequence GPGQQGPGGYGPGQQGPSGPGS linked by a peptide bond to the amino terminus of a second polypeptide having the amino acid sequence GAGAAAAAA(A) which is in turn linked by a peptide bond to the amino terminus of a third polypeptide having the amino acid sequence GPGGY, or tandem repeats of the amino acid sequence of said linked polypeptides.

49. The spider silk protein of any claim 30, 34, 37, and 46, comprising a first polypeptide having the amino acid sequence GPGQQGPGGYGPGQQGPSGPGS linked by a peptide bond to the amino terminus of a second polypeptide having the amino acid sequence GAGAAAAAA(A) which in turn is linked by a peptide bond to the amino terminus of a third polypeptide having the amino acid sequence of GPGQQ, or tandem repeats of the amino acid sequence of said linked polypeptides.

50. A silk protein of any claim 30 to 33, which comprises repeats of a polypeptide that is described by the general formula
[(v)(α)(β)]
wherein
v is a polypeptide of consensus sequence (A or G or S)GRGGL(A or G)GQ;
α is a polypeptide of consensus sequence GAGA(A or S or V)(A)m, wherein m is 4 to 6;
β is a polypeptide of consensus sequence GGAGQ(G or R or E)GY(G or R)GL(G or V)(G or S or N)QG.

51. The protein of claim 50, comprising repeating units selected from the group consisting of
QGAGAAAAAAGGAGQGGYGGLGGQG,
AGQGGYGGLGGQG,
AGQGAGAAAAAAAGGAGQGGYGGLGSQG,
AGRGGQGAGAAAAAAGGAGQGGYGGLGSQG,
AGRGGLGGQGAGAAAAAAAGGAGQGGYGGLGNQG,
AGRGGQGAAAAAAGGAGQGGYGGLGSQG,
AGRGGLGGQAGAAAAAAGGAGQGGYGGLGGQG,
AGQGGYGGLGSQG,
AGRGGLGGQGAGAAAAAAAGGAGQGGLGGQG,
AGQGAGASAAAAGGAGQGGYGGLGSQG,
AGRGGEGAGAAAAAAGGAGQGGYGGLGGQG,
AGQGGYGGLGSQG,
AGRGGLGGQGAGAAAAGGAGQGGLGGQG,
AGQGAGAAAAAAGGAGQGGYGGLGSQG,
AGRGGLGGQGAGAVAAAAAGGAGQGGYGGLGSQG,
AGRGGQGAGAAAAAAGGAGQRGYGGLGNQG,
AGRGGLGGQGAGAAAAAAAGGAGQGGYGGLGNQG,
AGRGGQGAAAAAGGAGQGGYGGLGSQG,
AGRGGQGAGAAAAAAVGAGQEGIRGQG,
AGQGGYGGLGSQG,
SGRGGLGGQGAGAAAAAAGGAGQGGLGGQG,
AGQGAGAAAAAAGGVRQGGYGGLGSQG,
AGRGGQGAGAAAAAAGGAGQGGYGGLGGQG,
VGRGGLGGQGAGAAAAGGAGQGGYGGVGSG,
ASAASAAASRLSS and mixtures thereof.

52. A purified silk protein of claim 30 and 34, which comprises repeats of a polypeptide which is described by the general formula
[(β)(α)(v)]
wherein
β is a polypeptide of consensus sequence GP(G or R)QQGPG(G or R)Y(G or A)PGQQG(P or L)SG(P or A)GS;
α is a polypeptide of consensus sequence A(A or S)AA(A or S)AA(A or S)A; and
v is a polypeptide of consensus sequence GPGQQG(P or L)GGY.

53. The protein of claim 52, comprising repeating units selected from the group consisting of
GPGQQGPGGYGPGQQGPSGPGSAAAAAAAAAAGPGGYGPGQQGPGGY,
GPGQQGPGRYGPGQQGPSGPGSAAAAAAGSGQQGPGGY,
GPRQQGPGGYGQGQQGPSGPGSAAAASAAASAESGQQGPGGYGPGQQGPGGY,
GPGQQGPGGYGPGQQGPSGPGSAAAAAAAASGPGQQGPGGY,
GPGQQGPGGYGPGQQGPSGPGSAAAAAAASGPGQQGPGGY,
GPGQQGPGGYGPGQQGLSGPGSAAAAAAA,
GPGQQGPGGYGPGQQGPSGPGSAAAAAAAAAGPGGY,
GPGQQGPGGYGPGQQGPSGAGSAAAAAAAGPGQQGLGGY,
GPGQQGPGGYGPGQQGPGGYGPGSASAAAAAA,
GPGQQGPGGYGPGQQGPSGPGSASAAAAAAAAGPGGY,
GPGQQGPGGYAPGQQGPSGPGSASAAAAAAAAGPGGY,
GPGQQGPGGYAPGQQGPSGPGSAAAAAAASAGPGGY and mixtures thereof.

## Patentansprüche

1. DNA-Molekül, das ein Spinnenseidenprotein codiert, welches repetitive Einheiten umfasst, umfassend eine durch die folgende Formel wiedergegebene Sequenz
[(A)ₘ(X)ₙ]ₚ
wobei m 4 bis 10 ist, n 10 bis 20 ist, p eine ganze Zahl aus 1 bis 100 ist und die Reste X gleich oder ungleich sind und ausgewählt aus der Gruppe, bestehend aus G, A, Q, Y und L, wobei mindestens 50% der X gleich G sind.

2. DNA nach Anspruch 1, die zudem variable Bereiche umfasst.

3. DNA nach Anspruch 1 oder 2, die ein Protein der Formel [(α)(β)]ₚ codiert, wobei α ein amorpher Bereich ist, der im gestreckten Zustand eine α-Helix bilden kann, β ein Bereich ist, der in gefalteter Konformation β-Faltblätter bilden kann, und p eine ganze Zahl von 25 bis 100 ist.

4. DNA nach Anspruch 1 oder 2, die ein Seidenprotein mit einem Molekulargewicht von weniger als 300000 Dalton oder ein Fragment oder eine Variante davon codiert, die repetitive α- und β-Bereiche umfassen, wobei α ein amorpher Bereich ist, der im gestreckten Zustand eine α-Helix bilden kann, und β ein Bereich ist, der β-Faltblätter bildet.

5. DNA nach Anspruch 1 oder 2, die ein Seidenprotein codiert, das die in Figur 6 gezeigte Aminosäuresequenz oder ein funktionelles Fragment davon umfasst.

6. DNA nach Anspruch 1 oder 2, die ein Seidenprotein codiert, das die in Figur 7 gezeigte Aminosäuresequenz oder ein funktionelles Fragment davon umfasst.

7. DNA nach Anspruch 1 oder 2, wobei p 15 bis 50 ist.

8. DNA nach Anspruch 1 oder 2, wobei p 25 bis 100 ist.

9. DNA nach Anspruch 3, wobei der α-Bereich ein alaninreicher Bereich ist, der 4 bis 10 A enthält.

10. DNA nach Anspruch 3, wobei der α-Bereich mindestens zu 50% Alanin enthält.

11. DNA nach Anspruch 1, die ein Protein codiert, das ein Polypeptid mit einer Aminosäuresequenz umfasst, umfassend repetitive Einheiten des Hexamers GGXGZG, wobei X und Z eine beliebige Aminosäure, aber am häufigsten Glutamin oder Alanin, sind, und repetitive Einheiten mit der Aminosäuresequenz (A)ₘGGAGQGGYGGLGGQG, wobei m 6 oder 7 ist.

12. DNA nach Anspruch 11, die ein Protein codiert, umfassend repetitive Einheiten mit der Aminosäuresequenz Ala-Gly-Arg-Gly-Gly-Xaa-Gly-Gly-Zaa-Gly-Ala-Gly-(Ala)ₘ-Gly-Gly-Ala-Gly-Gln-Gly-Gly-Baa-Gly-Gly-Leu-Gly-Gly-Gln-Gly, wobei m 6 oder 7 ist und Xaa, Zaa und Baa Leucin, Tyrosin oder Glutamin sind und Xaa nicht die gleiche Aminosäure wie Zaa ist.

13. DNA-Molekül nach Anspruch 12, wobei Xaa Glutamin ist.

14. DNA-Molekül nach Anspruch 1, das ein Protein codiert, das im Wesentlichen aus repetitiven Einheiten besteht, ausgewählt aus der Gruppe, bestehend aus
QGAGAAAAAAGGAGQGGYGGLGGQG,
AGQGGYGGLGGQG,
AGQGAGAAAAAAAGGAGQGGYGGLGSQG,
AGRGGQGAGAAAAAAGGAGQGGYGGLGSQG,
AGRGGLGGQGAGAAAAAAAGGAGQGGYGGLGNQG,
AGRGGQGAAAAAAGGAGQGGYGGLGSQG,
AGRGCLGGQAGAAAAAAGGAGQGGYGGLGGQG,
AGQGGYGGLGSQG,
AGRGGLGGQGAGAAAAAAAGGAGQGGLGGQG,
AGQGAGASAAAAGGAGQGGYGGLGSQG,
AGRGGEGAGAAAAAAGGAGQGGYGGLGGQG,
AGQGGYGGLGSQG,
AGRGGLGGQGAGAAAAGGAGQGGLGGQG,
AGQGAGAAAAAAGGAGQGGYGGLGSQG,
AGRGGLGGQGAGAVAAAAAGGAGQGGYGGLGSQG,
AGRGGQGAGAAAAAAGGAGQRGYGGLGNQG,
AGRGGLGGQGAGAAAAAAAGGAGQGGYGGLGNQG,
AGRGGQGAAAAAGGAGQGGYGGLGSQG,
AGRGGQGAGAAAAAAVGAGQEGIRGQG,
AGQGGYGGLGSQG,
SGRGGLGGQGAGAAAAAAGGAGQGGLGGQG,
AGQGAGAAAAAAGGVRQGGYGGLGSQG,
AGRGGQGAGAAAAAAGGAGQGGYGGLGGQG,
VGRGGLGGQGAGAAAAGGAGQGGYGGVGSG,
ASAASAAASRLSS und Gemischen davon

15. DNA-Molekül nach einem der Ansprüche 1 bis 14, wobei die repetitiven Einheiten durch eine variable Anzahl Alanin- oder Serinreste getrennt sind.

16. DNA, die ein Spinnenseidenprotein codiert, das repetitive Einheiten umfasst, umfassend eine durch die folgende Formel wiedergegebene Sequenz
[(A)ₘ(X)ₙ]ₚ
wobei m 4 bis 10 ist, n 10 bis 20 ist, p eine ganze Zahl aus 1 bis 100 ist und die Reste X, die gleich oder verschieden voneinander sein können, jeweils aus der Gruppe ausgewählt sind, bestehend aus P, G, A, Q, Y und L, wobei mindestens 50% der Reste X aus G bestehen.

17. DNA nach Anspruch 16, wobei p 15 bis 50 ist.

18. DNA nach Anspruch 16, wobei p 25 bis 100 ist.

19. DNA nach Anspruch 16, die Protein codiert, das im Wesentlichen aus repetitiven Einheiten besteht, ausgewählt aus der Gruppe, bestehend aus
GPGQQGPGGYGPGQQGPSGPGSAAAAAAAAAAGPGGYGPGQQGPGGY,
GPGQQGPGRYGPGQQGPSGPGSAAAAAAGSGQQGPGGY,
GPRQQGPGGYGQGQQGPSGPGSAAAASAAASAESGQQGPGGYGPGQQGPGGY,
GPGQQGPGGYGPGQQGPSGPGSAAAAAAAASGPGQQGPGGY,
GPGQQGPGGYGPGQQGPSGPGSAAAAAAASGPGQQGPGGY,
GPGQQGPGGYGPGQQGLSGPGSAAAAAAA,
GPGQQGPGGYGPGQQGPSGPGSAAAAAAAAAGPGGY,
GPGQQGPGGYGPGQQGPSGAGSAAAAAAAGPGQQGLGGY,
GPGQQGPGGYGPGQQGPGGYGPGSASAAAAAA,
GPGQQGPGGYGPGQQGPSGPGSASAAAAAAAAGPGGY,
GPGQQGPGGYAPGQQGPSGPGSASAAAAAAAAGPGGY,
GPGQQGPGGYAPGQQGPSGPGSAAAAAAASAGPGGY
und Gemischen davon.

20. DNA nach einem der vorhergehenden Ansprüche 1 bis 21, wobei das Seidenprotein ein Molekulargewicht von 100000 bis 300000 Dalton hat.

21. Plasmid PLM-4, das im ATCC-Depot Nr. 68567 enthalten ist.

22. Plasmid pMB-2, das im ATCC-Depot Nr. 68568 enthalten ist.

23. Replizierbarer Vektor, der eine DNA nach einem der Ansprüche 1 bis 22 enthält und ein Spinnenseidenprotein oder ein Fragment oder eine Variante davon exprimieren kann, wenn er in einen geeigneten Wirt eingebracht wird.

24. Lebende Zelle oder Mikroorganismus, die/der den Vektor nach einem der Ansprüche 20 bis 23 enthält.

25. Lebende Zelle oder Mikroorganismus nach Anspruch 24, die/der ein Bakterium, vorzugsweise *Escherichia coli*, ist.

26. Transformierte Zelle oder transformierter Mikroorganismus, die/der die DNA nach einem der Ansprüche 1 bis 23 enthält, die ein Spinnenseidenprotein oder ein funktionelles Fragment davon codiert, wobei die Zelle oder der Mikroorganismus das Spinnenseidenprotein exprimieren kann.

27. Verfahren zur Herstellung rekombinanter Spinnenseidenproteine oder funktioneller Fragmente davon, umfassend die Verwendung einer DNA oder eines Vektors nach einem der Ansprüche 1 bis 23 und/oder eine lebende Zelle oder einen lebenden Mikroorganismus nach einem der Ansprüche 24 bis 26.

28. Verfahren zur Herstellung einer Faser aus Spinnenseidenproteinen oder funktionellen Fragmenten davon, umfassend ein Verfahren nach Anspruch 27 und/oder die Verwendung von Proteinen, hergestellt durch ein Verfahren nach Anspruch 27.

29. Verfahren zur Herstellung von rekombinantem Spinnenseidenprotein, umfassend das Züchten der transformierten Zelle oder des transformierten Mikroorganismus nach einem der Ansprüche 24 bis 26.

30. Funktionelles Fragment eines Spinnenseidenproteins, umfassend repetitive α- und β-Bereiche, wobei α ein amorpher Bereich ist, der im gestreckten Zustand eine α-Helix bilden kann, und β ein Bereich ist, der β-Faltblätter bildet, das im Wesentlichen frei von natürlichen Spinnenseidenproteinen ist, wobei das Fragment oder die Variante durch ein Verfahren nach Anspruch 27 erhältlich ist.

31. Gereinigtes Spinnenseidenprotein, das im Wesentlichen ein einzelnes Protein ist und repetitive Einheiten umfasst, die eine durch die folgende Formel wiedergegebene Sequenz enthalten
[(A)ₘ(X)ₙ]ₚ
wobei m 4 bis 10 ist, n 10 bis 20 ist, p eine ganze Zahl aus 1 bis 100 ist und die Reste X, die gleich oder verschieden voneinander sein können, jeweils aus der Gruppe ausgewählt sind, bestehend aus G, A, Q, Y und L, wobei mindestens 50% der Reste X aus G bestehen.

32. Protein nach Anspruch 31, wobei für die repetitiven Einheiten "p" in der Formel [(A)ₘ(X)ₙ]ₚ m und n jeweils gleich sind und vorzugsweise 10 sind.

33. Spinnenseidenprotein mit repetitiven Einheiten nach Anspruch 1, die eine Sequenz enthalten, die sich durch die folgende allgemeine Formel wiedergeben lässt
[(α)(β)]ₚ
wobei α ein amorpher Bereich ist, der im gestreckten Zustand eine α-Helix bilden kann, β ein β-kristalliner Bereich ist und p eine ganze Zahl von 1 bis 100 ist, das im Wesentlichen frei von einem Spinnenseidenprotein ist, das die in Figur 7 gezeigte Aminosäuresequenz umfasst.

34. Spinnenseidenprotein mit repetitiven Einheiten nach Anspruch 16, die eine Sequenz enthalten, die sich durch die folgende allgemeine Formel wiedergeben lässt
[(β)(α)]ₚ
wobei α ein amorpher Bereich ist, der im gestreckten Zustand eine α-Helix bilden kann, β ein Bereich ist, der eine β-Faltblatt-ähnliche Struktur bildet, und p eine ganze Zahl von 1 bis 100 ist, das im Wesentlichen frei von einem Spinnenseidenprotein ist, das die in Figur 6 gezeigte Aminosäuresequenz umfasst.

35. Verfahren zur Herstellung eines Seidenproteingemischs, umfassend das Mischen des Seidenproteins nach Anspruch 33 mit dem Seidenprotein nach Anspruch 34.

36. Spinnenseidenprotein mit repetitiven Einheiten nach Anspruch 1, die eine Sequenz enthalten, die sich durch die folgende allgemeine Formel wiedergeben lässt
[(α)(β)]ₚ
wobei α ein amorpher Bereich ist, der im gestreckten Zustand eine α-Helix bilden kann, β ein β-kristalliner Bereich ist und p eine ganze Zahl von 1 bis 100 ist, wobei für jede repetitive Einheit "p" in der obigen Formel die Bereiche α und β gleich sind.

37. Spinnenseidenprotein mit repetitiven Einheiten nach Anspruch 16, die eine Sequenz enthalten, die sich durch die folgende allgemeine Formel wiedergeben lässt
[(β)(α)]ₚ
wobei α ein amorpher Bereich ist, der im gestreckten Zustand eine α-Helix bilden kann, β ein Bereich aus verbundenen β-Windungen ist und eine β-Faltblatt-ähnliche Struktur bildet und p eine ganze Zahl von 1 bis 100 ist, wobei für jede repetitive Einheit "p" in der obigen Formel die Bereiche α und β gleich sind.

38. Spinnenseidenprotein nach einem der Ansprüche 30 bis 37, das repetitive α- und β-Einheiten besitzt, wobei eine α-Einheit ein amorpher Bereich ist, der im gestreckten Zustand eine α-Helix bilden kann, und eine β-Einheit ein Bereich ist, der β-Faltblätter bilden kann, und das Protein ein Molekulargewicht von 16000 bis 300000 Dalton hat.

39. Spinnenseidenprotein nach einem der Ansprüche 30 bis 33, umfassend ein Polypeptid mit einer Aminosäuresequenz, die repetitive Einheiten des Hexamers GGXGZG umfasst, wobei X und Z eine beliebige Aminosäure, aber am häufigsten Glutamin oder Alanin sind.

40. Seidenprotein nach einem der Ansprüche 30 bis 33, wobei X und Z Glutamin oder Alanin sind.

41. Seidenprotein nach einem der Ansprüche 30 bis 33, wobei die repetitiven Einheiten durch eine variable Anzahl Alanin- oder Serinreste getrennt sind.

42. Seidenprotein nach Anspruch 39, das zudem repetitive Einheiten mit der Sequenz (A)ₘGGAGQGGYGGLGGQG umfasst, wobei m 6 oder 7 ist.

43. Spinnenseidenprotein nach einem der Ansprüche 30 bis 33, umfassend ein Polypeptid mit der Aminosäuresequenz (A)ₘGGAGQGGYGGLGGQG, wobei m 6 oder 7 ist, oder Tandemrepeats dieser Aminosäuresequenz.

44. Spinnenseidenprotein nach Anspruch 33, umfassend ein Polypeptid mit der Aminosäuresequenz Ala-Gly-Arg-Gly-Gly-Xaa-Gly-Gly-Zaa-Gly-Ala-Gly-(Ala)ₘ-Gly-Gly-Ala-Gly-Glu-Gly Gly-Baa-Gly-Gly-Leu-Gly-Gly-Glu-Gly, wobei m 6 oder 7 ist und Xaa, Zaa und Baa Leucin, Tyrosin oder Glutamin sind und Xaa nicht die gleiche Aminosäure wie Zaa ist.

45. Spinnenseidenprotein nach Anspruch 33, umfassend ein Polypeptid mit der Aminosäuresequenz GAAAAAA (A), das über eine Peptidbindung an den Aminoterminus eines Polypeptids mit der Aminosäuresequenz GGAGQGGYGGLGGQG, wobei Y Tyrosin ist, gebunden ist.

46. Seidenprotein nach einem der Ansprüche 30, 34 und 37, umfassend ein Polypeptid mit einer Aminosäuresequenz, die repetitive Einheiten mit der Aminosäuresequenz GPGQQGPGYYGGQQGPSGPGS umfasst.

47. Spinnenseidenprotein nach einem der Ansprüche 30, 34, 37 und 46, umfassend ein Polypeptid mit der Aminosäuresequenz GPGQQGPGGYGPGQQGPSGPGS, das über eine Peptidbindung an den Aminoterminus eines Polypeptids mit der Aminosäuresequenz GAGAAAAAA(A) gebunden ist, oder Tandemrepeats der Aminosäuresequenz der verbundenen Polypeptide.

48. Spinnenseidenprotein nach einem der Ansprüche 30, 34, 37 und 46, umfassend ein erstes Polypeptid mit der Aminosäuresequenz GPGQQGPGGYGPGQQGPSGPGS, das über eine Peptidbindung an den Aminoterminus eines zweiten Polypeptids mit der Aminosäuresequenz GAGAAAAAA(A) gebunden ist, das wiederum über eine Peptidbindung an den Aminoterminus eines dritten Polypeptids mit der Aminosäuresequenz GPGGY gebunden ist, oder Tandemrepeats der Aminosäuresequenz der verbundenen Polypeptide.

49. Spinnenseidenprotein nach einem der Ansprüche 30, 34, 37 und 46, umfassend ein erstes Polypeptid mit der Aminosäuresequenz GPGQQGPGGYGPGQQGPSGPGS, das über eine Peptidbindung an den Aminoterminus eines zweiten Polypeptids mit der Aminosäuresequenz GAGAAAAAA(A) gebunden ist, das wiederum über eine Peptidbindung an den Aminoterminus eines dritten Polypeptids mit der Aminosäuresequenz GPGQQ gebunden ist, oder Tandemrepeats der Aminosäuresequenz der verbundenen Polypeptide.

50. Seidenprotein nach einem der Ansprüche 30 bis 33, umfassend repetitive Einheiten eines Polypeptids, das beschrieben wird durch die allgemeine Formel
[(v)(α)(β)]
wobei
v ein Polypeptid der Konsensussequenz (A oder G oder S)GRGGL(A oder G)GQ ist;
α ein Polypeptid der Konsensussequenz GAGA(A oder S oder V)(A)ₘ ist, wobei m 4 bis 6 ist;
β ein Polypeptid der Konsensussequenz GGAGQ(G oder R oder E)GY(G oder R)GL(G oder V) (G oder S oder N)QG ist.

51. Protein nach Anspruch 50, umfassend repetitive Einheiten, ausgewählt aus der Gruppe, bestehend aus
QGAGAAAAAAGGAGQGGYGGLGGQG,
AGQGGYGGLGGQG,
AGQGAGAAAAAAAGGAGQGGYGGLGSQG,
AGRGGQGAGAAAAAAGGAGQGGYGGLGSQG,
AGRGGLGGQGAGAAAAAAAGGAGQGGYGGLGNQG,
AGRGGQGAAAAAAGGAGQGGYGGLGSQG,
AGRGGLGGQAGAAAAAAGGAGQGGYGGLGGQG,
AGQGGYGGLGSQG,
AGRGGLGGQGAGAAAAAAAGGAGQGGLGGQG,
AGQGAGASAAAAGGAGQGGYGGLGSQG,
AGRGGEGAGAAAAAAGGAGQGGYGGLGGQG,
AGQGGYGGLGSQG,
AGRGGLGGQGAGAAAAGGAGQGGLGGQG,
AGQGAGAAAAAAGGAGQGGYGGLGSQG,
AGRGGLGGQGAGAVAAAAAGGAGQGGYGGLGSQG,
AGRGGQGAGAAAAAAGGAGQRGYGGLGNQG,
AGRGGLGGQGAGAAAAAAAGGAGQGGYGGLGNQG,
AGRGGQGAAAAAGGAGQGGYGGLGSQG,
AGRGGQGAGAAAAAAVGAGQEGIRGQG,
AGQGGYGGLGSQG,
SGRGGLGGQGAGAAAAAAGGAGQGGLGGQG,
AGQGAGAAAAAAGGVRQGGYGGLGSQG,
AGRGGQGAGAAAAAAGGAGQGGYGGLGGQG,
VGRGGLGGQGAGAAAAGGAGQGGYGGVGSG,
ASAASAAASRLSS
und Gemischen davon.

52. Gereinigtes Seidenprotein nach Anspruch 30 und 34, umfassend repetitive Einheiten eines Polypeptids, das beschrieben wird durch die allgemeine Formel
[(β)(α)(v)]
wobei
β ein Polypeptid der Konsensussequenz GP (G oder R) QQGPG (G oder R)Y(G oder A)PGQQG(P oder L)SG(P oder A)GS ist;
α ein Polypeptid der Konsensussequenz A(A oder S)AA(A oder S)AA(A oder S)(A) ist, und
v ein Polypeptid der Konsensussequenz GPGQQG(P oder L)GGY ist.

53. Protein nach Anspruch 52, umfassend repetitive Einheiten, ausgewählt aus der Gruppe, bestehend aus
GPGQQGPGGYGPGQQGPSGPGSAAAAAAAAAAGPGGYGPGQQGPGGY,
GPGQQGPGRYGPGQQGPSGPGSAAAAAAGSGQQGPGGY,
GPRQQGPGGYGQGQQGPSGPGSAAAASAAASAESGQQGPGGYGPGQQGPGGY,
GPGQQGPGGYGPGQQGPSGPGSAAAAAAAASGPGQQGPGGY,
GPGQQGPGGYGPGQQGPSGPGSAAAAAAASGPGQQGPGGY,
GPGQQGPGGYGPGQQGLSGPGSAAAAAAA,
GPGQQGPGGYGPGQQGPSGPGSAAAAAAAAAGPGGY,
GPGQQGPGGYGPGQQGPSGAGSAAAAAAAGPGQQGLGGY,
GPGQQGPGGYGPGQQGPGGYGPGSASAAAAAA,
GPGQQGPGGYGPGQQGPSGPGSASAAAAAAAAGPGGY,
GPGQQGPGGYAPGQQGPSGPGSASAAAAAAAAGPGGY,
GPGQQGPGGYAPGQQGPSGPGSAAAAAAASAGPGGY
und Gemischen davon.

## Revendications

1. Molécule d'ADN qui code pour une protéine de soie d'araignée comprenant des unités répétées, dans laquelle lesdites unités répétées sont composées d'une séquence représentée par la formule :
[(A)ₘ (X)ₙ]ₚ
dans laquelle m est 4 à 10, n est 10 à 20, p est un entier de 1 à 100 et chacun des X, qui peuvent être identiques ou différents, est choisi dans le groupe consistant en G, A, Q, Y et L, où au moins 50% des X sont G.

2. ADN suivant la revendication 1, qui comprend de plus des régions variables.

3. ADN suivant les revendications 1 ou 2, qui code pour une protéine de formule [(α) (β)]ₚ, dans laquelle α est une région amorphe qui peut former une hélice α lors qu'elle est étirée et β est une région qui peut former des feuillets β lorsqu'elle est en conformation repliée, et p est un entier de 25 à 100.

4. ADN suivant les revendications 1 ou 2, qui code pour une protéine de soie ayant un poids moléculaire inférieur à 300 000 daltons, ou un fragment ou un variant de celle-ci, qui comprend des régions α et β répétées, où α est une région amorphe qui forme une hélice α lorsqu'elle est étirée et β est une région qui forme des feuillets β.

5. ADN suivant les revendications 1 ou 2, qui code pour une protéine de soie comprenant la séquence d'acides aminés telle qu'elle est représentée dans la figure 6 ou un fragment fonctionnel de celle-ci.

6. ADN suivant les revendications 1 ou 2, qui code pour une protéine de soie comprenant la séquence d'acides aminés telle qu'elle est représentée dans la figure 7 ou un fragment fonctionnel de celle-ci.

7. ADN suivant les revendications 1 ou 2, dans lequel p est 15 à 50.

8. ADN suivant les revendications 1 ou 2, dans lequel p est 25 à 100.

9. ADN suivant la revendication 3, dans lequel la région α est une région riche en alanine qui contient 4 à 10 A.

10. ADN suivant la revendication 3, dans lequel la région α contient au moins 50% d'alanine.

11. ADN suivant la revendication 1, qui code pour une protéine comprenant un polypeptide ayant une séquence d'acides aminés qui comprend des unités répétées de l'hexamère GGXGZG, dans lequel X et Z sont un quelconque acide aminé mais sont le plus souvent la glutamine ou l'alanine, et des unités répétées ayant la séquence d'acides aminés (A)ₘGGAGQGGYGGLGGQG, dans laquelle m est 6 ou 7.

12. Molécule d'ADN suivant la revendication 1, qui code pour une protéine comprenant des unités répétées ayant la séquence d'acides aminés Ala-Gly-Arg-Gly-Gly-Xaa-Gly-Gly-Zaa-Gly-Ala-Gly-(Ala)ₘ-Gly-Gly-Ala-Gly-Gln-Gly-Gly-Baa-Gly-Gly-Leu-Gly-Gly-Gln-Gly, dans laquelle m est 6 ou 7 et dans laquelle Xaa, Zaa et Baa sont la leucine, la tyrosine ou la glutamine, et Xaa n'est pas le même acide aminé que Zaa.

13. Molécule d'ADN suivant la revendication 12, dans laquelle Xaa est la glutamine.

14. Molécule d'ADN suivant la revendication 1, qui code pour une protéine consistant essentiellement en unités répétées choisies dans le groupe consistant en :
QGAGAAAAAAGGAGQGGYGGLGGQG,
AGQGGYGGLGGQG,
AGQGAGAAAAAAAGGAGQGGYGGLGSQG,
AGRGGQGAGAAAAAAGGAGQGGYGGLGSQG,
AGRGGLGGQGAGAAAAAAAGGAGQGGYGGLGNQG,
AGRGGQGAAAAAAGGAGQGGYGGLGSQG,
AGRGGLGGQAGAAAAAAGGAGQGGYGGLGGQG,
AGQGGYGGLGSQG,
AGRGGLGGQGAGAAAAAAAGGAGQGGLGGQG,
AGQGAGASAAAAGGAGQGGYGGLGSQG,
AGRGGEGAGAAAAAAGGAGQGGYGGLGGQG,
AGQGGYGGLGSQG,
AGRGGLGGQGAGAAAAGGAGQGGLGGQG,
AGQGAGAAAAAAGGAGQGGYGGLGSQG,
AGRGGLGGQGAGAVAAAAAGGAGQGGYGGLGSQG,
AGRGGQGAGAAAAAAGGAGQRGYGGLGNQG,
AGRGGLGGQGAGAAAAAAAGGAGQGGYGGLGNQG,
AGRGGQGAAAAAGGAGQGGYGGLGSQG,
AGRGGQGAGAAAAAAVGAGQEGIRGQG,
AGQGGYGGLGSQG,
SGRGGLGGQGAGAAAAAAGGAGQGGLGGQG,
AGQGAGAAAAAAGGVRQGGYGGLGSQG,
AGRGGQGAGAAAAAAGGAGQGGYGGLGGQG,
VGRGGLGGQGAGAAAAGGAGQGGYGGVGSG,
ASAASAAASRLSS
et leurs mélanges.

15. Molécule d'ADN suivant l'une quelconque des revendications 1 à 14, dans laquelle les unités répétées sont séparées par des nombres variables de résidus alanine ou sérine.

16. ADN qui code pour une protéine de soie d'araignée comprenant des unités répétées, dans laquelle lesdites unités répétées sont composées d'une séquence représentée par la formule :
[(A)ₘ (X)ₙ]ₚ
dans laquelle m est 4 à 10, n est 10 à 20, p est un entier de 1 à 100 et chacun des X, qui peuvent être identiques ou différents, est choisi dans le groupe consistant en P, G, A, Q, Y et L, où au moins 50% des X sont G.

17. ADN suivant la revendication 16, dans lequel p est 15 à 50.

18. ADN suivant la revendication 16, dans lequel p est 25 à 100.

19. ADN suivant la revendication 16, qui code pour une protéine consistant essentiellement en unités répétées choisies dans le groupe consistant en :
GPGQQGPGGYGPGQQGPSGPGSAAAAAAAAAAGPGGYGPGQQGPGGY,
GPGQQGPGRYGPGQQGPSGPGSAAAAAAGSGQQGPGGY,
GPRQQGPGGYGQGQQGPSGPGSAAAASAAASAESGQQGPGGYGPGQQGPGGY,
GPGQQGPGGYGPGQQGPSGPGSAAAAAAAASGPGQQGPGGY,
GPGQQGPGGYGPGQQGPSGPGSAAAAAAASGPGQQGPGGY,
GPGQQGPGGYGPGQQGLSGPGSAAAAAAA,
GPGQQGPGGYGPGQQGPSGPGSAAAAAAAAAGPGGY,
GPGQQGPGGYGPGQQGPSGAGSAAAAAAAGPGQQGLGGY,
GPGQQGPGGYGPGQQGPGGYGPGSASAAAAAA,
GPGQQGPGGYGPGQQGPSGPGSASAAAAAAAAGPGGY,
GPGQQGPGGYAPGQQGPSGPGSASAAAAAAAAGPGGY,
GPGQQGPGGYAPGQQGPSGPGSAAAAAAASAGPGGY
et leurs mélanges.

20. ADN suivant l'une quelconque des revendications précédentes 1 à 21, dans lequel ladite protéine de soie est une protéine ayant un poids moléculaire compris entre 100 000 et 300 000 daltons.

21. Plasmide PLM-4 qui est contenu dans le dépôt ATCC No 68567.

22. Plasmide pMB-2 qui est contenu dans le dépôt ATCC No 68568.

23. Vecteur réplicable contenant un ADN suivant l'une quelconque des revendications 1 à 22, qui est capable d'exprimer une protéine de soie d'araignée ou un fragment ou variant de celle-ci, lorsqu'il est introduit dans un hôte convenable.

24. Cellule ou microorganisme vivant contenant le vecteur suivant l'une quelconque des revendications 20 à 23.

25. Cellule ou microorganisme vivant suivant la revendication 24, qui est une bactérie, de préférence *Escherichia coli*.

26. Cellule ou microorganisme transformé contenant l'ADN suivant l'une quelconque des revendications 1 à 23, qui code pour une protéine de soie d'araignée ou un fragment fonctionnel de celle-ci, laquelle cellule ou lequel microorganisme est capable d'exprimer une protéine de soie d'araignée.

27. Procédé pour préparer des protéines recombinantes de soie d'araignée ou des fragments fonctionnels de celles-ci, comprenant l'utilisation d'un ADN ou d'un vecteur suivant l'une quelconque des revendications 1 à 23 et/ou d'une cellule ou d'un microorganisme vivant suivant l'une quelconque des revendications 24 à 26.

28. Procédé pour préparer une fibre de protéines recombinantes de soie d'araignée ou de fragments fonctionnels de celles-ci, comprenant un procédé suivant la revendication 27 et/ou l'utilisation de protéines préparées par un procédé suivant la revendication 27.

29. Méthode pour produire une protéine recombinante de soie d'araignée, qui comprend la culture de la la cellule ou du microorganisme transformé suivant l'une quelconque des revendications 24 à 26.

30. Fragment fonctionnel de protéine de soie d'araignée, qui comprend des régions α et β, dans lequel α est une région amorphe qui forme une hélice α lorsqu'elle est étirée et β est une région qui forme des feuillets β et qui est pratiquement dépourvu de protéines naturelles de soie d'araignée, lequel fragment ou variant peut être obtenu par un procédé suivant la revendication 27.

31. Protéine purifiée de soie d'araignée, qui est essentiellement une protéine unique qui comprend des unités répétées qui contiennent une séquence représentée par la formule:
[(A)ₘ (X)ₙ]ₚ
dans laquelle m est 4 à 10, n est 10 à 20, p est un entier de 1 à 100 et chacun des X, qui peuvent être identiques ou différents, est choisi dans le groupe consistant en G, A, Q, Y et L, où au moins 50% des X sont G.

32. Protéine suivant la revendication 31, dans laquelle pour chaque répétition "p" dans la formule [(A)ₘ (X)ₙ]ₚ, chacun des m et n est identiques, de préférence 10.

33. Protéine de soie d'araignée comprenant des unités répétées, suivant la revendication 1, qui contiennent une séquence qui peut être représentée par la formule générale suivante :
[(α) (β)]ₚ
dans laquelle α est une région amorphe qui peut former une hélice α lorsqu'elle est étirée, β est une région cristalline β et p est un entier de 1 à 100, qui est pratiquement dépourvue d'une protéine de soie d'araignée comprenant la séquence d'acides aminés représentée dans la figure 7.

34. Protéine de soie d'araignée comprenant des unités répétées, suivant la revendication 16, qui contiennent une séquence qui peut être représentée par la formule générale suivante :
[(β) (α)]ₚ
dans laquelle α est une région amorphe qui peut former une hélice α lorsqu'elle est étirée, β est une région qui forme une structure semblable à un feuillet β et p est un entier de 1 à 100, qui est pratiquement dépourvue d'une protéine de soie d'araignée comprenant la séquence d'acides aminés représentée dans la figure 6.

35. Procédé pour préparer un mélange de protéines de soie, qui comprend le mélange de la protéine de soie suivant la revendication 33 avec la protéine de soie suivant la revendication 34.

36. Protéine de soie d'araignée comprenant des unités répétées, suivant la revendication 1, qui contiennent une séquence qui peut être représentée par la formule générale suivante :
[(α) (β)]ₚ
dans laquelle α est une région amorphe qui peut former une hélice α lorsqu'elle est étirée, β est une région cristalline β et p est un entier de 1 à 100, dans laquelle pour chaque "p" répété dans la formule ci-dessus, les régions α et β sont les mêmes.

37. Protéine de soie d'araignée comprenant des unités répétées, suivant la revendication 16, qui contiennent une séquence qui peut être représentée par la formule générale suivante :
[(β) (α)]ₚ
dans laquelle α est une région amorphe qui peut former une hélice α lorsqu'elle est étirée, β est une région liée à tour β qui forme une structure ressemblant à un feuillet β et p est un entier de 1 à 100, dans laquelle pour chaque "p" répété dans la formule ci-dessus, les régions α et β sont les mêmes.

38. Protéine de soie d'araignée suivant l'une quelconque des revendications 30 à 37, qui a des unités répétées α et β, dans laquelle une unité α est une région amorphe qui peut former une hélice α lorsqu'elle est étirée et une unité β est une région qui peut former des feuillets β, et où ladite protéine a un poids moléculaire compris dans la gamme de 16 000 à 300 000 daltons.

39. Protéine de soie d'araignée suivant l'une quelconque des revendications 30 à 33, qui comprend un polypeptide ayant une séquence d'acides aminés qui comprend des unités répétées de l'hexamère GGXGZG, dans lequel X et Z sont un quelconque acide aminé mais sont le plus souvent la glutamine ou l'alanine.

40. Protéine de soie suivant l'une quelconque des revendications 30 à 33, dans laquelle X et Z sont la glutamine ou l'alanine.

41. Protéine de soie suivant l'une quelconque des revendications 30 à 33, dans laquelle les unités répétées sont séparées par des nombres variables de résidus alanine ou sérine.

42. Protéine de soie suivant la revendication 39, qui comprend de plus des unités répétées ayant la séquence (A)ₘGGAGQGGYGGLGGQG, dans laquelle m est 6 ou 7.

43. Protéine de soie d'araignée suivant l'une quelconque des revendications 30 à 33, qui comprend un polypeptide ayant la séquence d'acides aminés (A)ₘGGAGQGGYGGLGGQG, dans laquelle m est 6 ou 7, ou des unités répétées en tandem de cette séquence d'acides amines.

44. Protéine de soie d'araignée suivant la revendication 33, qui comprend un polypeptide ayant la séquence d'acides aminés Ala-Gly-Arg-Gly-Gly-Xaa-Gly-Gly-Zaa-Gly-Ala-Gly-(Ala)ₘ-Gly-Gly-Ala-Gly-Glu-Gly-Gly-Baa-Gly-Gly-Leu-Gly-Gly-Glu-Gly, dans laquelle m est 6 ou 7 et dans laquelle Xaa, Zaa et Baa sont la leucine, la tyrosine ou la glutamine, et Xaa n'est pas le même acide aminé que Zaa.

45. Protéine de soie d'araignée suivant la revendication 33, qui comprend un polypeptide ayant la séquence d'acides aminés GAAAAAA(A) lié par une liaison peptidique à l'amino terminal d'un polypeptide ayant la séquence d'acides aminés GGAGQGGYGGLGGQG, dans laquelle Y est la tyrosine.

46. Protéine de soie suivant l'une quelconque des revendications 30, 34 et 37, qui comprend un polypeptide ayant une séquence d'acides aminés comprenant des unités répétées ayant la séquence d'acides aminés GPGQQGPGYYGGQQGPSGPGS.

47. Protéine de soie d'araignée suivant l'une quelconque des revendications 30, 34, 37 et 46, qui comprend un polypeptide ayant la séquence d'acides aminés GPGQQGPGGYGPGQQGPSGPGS lié par une liaison peptidique à l'amino terminal d'un polypeptide ayant la séquence d'acides aminés GAGAAAAAA(A), ou des unités répétées en tandem de la séquence d'acides aminés desdits polypeptides liés.

48. Protéine de soie d'araignée suivant l'une quelconque des revendications 30, 34, 37 et 46, qui comprend un premier polypeptide ayant la séquence d'acides aminés GPGQQGPGGYGPGQQGPSGPGS lié par une liaison peptidique à l'amino terminal d'un deuxième polypeptide ayant la séquence d'acides aminés GAGAAAAAA(A) qui est à son tour lié par une liaison peptidique à l'amino terminal d'un troisième polypeptide ayant la séquence d'acides aminés GPGGY, ou des unités répétées en tandem de la séquence d'acides aminés desdits polypeptides liés.

49. Protéine de soie d'araignée suivant l'une quelconque des revendications 30, 34, 37 et 46, qui comprend un premier polypeptide ayant la séquence d'acides aminés GPGQQGPGGYGPGQQGPSGPGS lié par une liaison peptidique à l'amino terminal d'un deuxième polypeptide ayant la séquence d'acides aminés GAGAAAAAA(A) qui est à son tour lié par une liaison peptidique à l'amino terminal d'un troisième polypeptide ayant la séquence d'acides aminés GPGQQ, ou des unités répétées en tandem de la séquence d'acides aminés desdits polypeptides liés.

50. Protéine de soie suivant l'une quelconque des revendications 30 à 33, qui comprend des unités répétées d'un polypeptide qui est décrit par la formule générale :
[(v) (α) (β)]
dans laquelle :
v est un polypeptide de séquence consensus (A ou G ou S)GRGGL(A ou G)GQ;
α est un polypeptide de séquence consensus GAGA(A ou S ou V) (A)ₘ, dans laquelle m est 4 à 6 ;
β est un polypeptide de séquence consensus GGAGQ(G ou R ou E)GY(G ou R)GL(G ou V)(G ou S ou N)QG.

51. Protéine suivant la revendication 50, comprenant des unités répétées choisies dans le groupe consistant en :
QGAGAAAAAAGGAGQGGYGGLGGQG,
AGQGGYGGLGGQG,
AGQGAGAAAAAAAGGAGQGGYGGLGSQG,
AGRGGQGAGAAAAAAGGAGQGGYGGLGSQG,
AGRGGLGGQGAGAAAAAAAGGAGQGGYGGLGNQG,
AGRGGQGAAAAAAGGAGQGGYGGLGSQG,
AGRGGLGGQAGAAAAAAGGAGQGGYGGLGGQG,
AGQGGYGGLGSQG,
AGRGGLGGQGAGAAAAAAAGGAGQGGLGGQG,
AGQGAGASAAAAGGAGQGGYGGLGSQG,
AGRGGEGAGAAAAAAGGAGQGGYGGLGGQG,
AGQGGYGGLGSQG,
AGRGGLGGQGAGAAAAGGAGQGGLGGQG,
AGQGAGAAAAAAGGAGQGGYGGLGSQG,
AGRGGLGGQGAGAVAAAAAGGAGQGGYGGLGSQG,
AGRGGQGAGAAAAAAGGAGQRGYGGLGNQG,
AGRGGLGGQGAGAAAAAAAGGAGQGGYGGLGNQG,
AGRGGQGAAAAAGGAGQGGYGGLGSQG,
AGRGGQGAGAAAAAAVGAGQEGIRGQG,
AGQGGYGGLGSQG,
SGRGGLGGQGAGAAAAAAGGAGQGGLGGQG,
AGQGAGAAAAAAGGVRQGGYGGLGSQG,
AGRGGQGAGAAAAAAGGAGQGGYGGLGGQG,
VGRGGLGGQGAGAAAAGGAGQGGYGGVGSG,
ASAASAAASRLSS
et leurs mélanges.

52. Protéine de soie purifiée suivant les revendications 30 et 34, qui comprend des unités répétées d'un polypeptide qui est décrit par la formule générale :
[(β) (α) (v)]
dans laquelle :
β est un polypeptide de séquence consensus GP(G ou R)QQGPG(G ou R)Y(G ou A)PGQQG(P ou L)SG(P ou A)GS;
α est un polypeptide de séquence consensus A(A ou S)AA(A ou S)AA(A ou S)A ; et
v es t un polypeptide de séquence consensus GPGQQG(P ou L)GGY.

53. Protéine suivant la revendication 52, comprenant des unités répétées choisies dans le groupe consistant en :
GPGQQGPGGYGPGQQGPSGPGSAAAAAAAAAAGPGGYGPGQQGPGGY,
GPGQQGPGRYGPGQQGPSGPGSAAAAAAGSGQQGPGGY,
GPRQQGPGGYGQGQQGPSGPGSAAAASAAASAESGQQGPGGYGPGQQGPGGY,
GPGQQGPGGYGPGQQGPSGPGSAAAAAAAASGPGQQGPGGY,
GPGQQGPGGYGPGQQGPSGPGSAAAAAAASGPGQQGPGGY,
GPGQQGPGGYGPGQQGLSGPGSAAAAAAA,
GPGQQGPGGYGPGQQGPSGPGSAAAAAAAAAGPGGY,
GPGQQGPGGYGPGQQGPSGAGSAAAAAAAGPGQQGLGGY,
GPGQQGPGGYGPGQQGPGGYGPGSASAAAAAA,
GPGQQGPGGYGPGQQGPSGPGSASAAAAAAAAGPGGY,
GPGQQGPGGYAPGQQGPSGPGSASAAAAAAAAGPGGY,
GPGQQGPGGYAPGQQGPSGPGSAAAAAAASAGPGGY
et leurs mélanges.
